# EUROPEAN PATENT SPECIFICATION

(11) **EP 1 999 269 B1**
(45) Date of publication and mention of the grant of the patent: **10.12.2014**
(21) Application number: 07752648.1
(22) Date of filing: 08.03.2007
(51) Int. Cl.: A61K 38/17, A61K 47/48, G01N 33/574

(54) **SOLUBLE MONOMERIC EPHRIN A1**
LÖSLICHES MONOMERES EPHRIN-A1
ÉPHRINE-A1 MONOMÈRE SOLUBLE

(30) Priority: 08.03.2006 US 780241 P
(43) Date of publication of application: 10.12.2008
(73) Proprietor: Wake Forest University Health Sciences, Winston-Salem, North Carolina 27101 (US)
(72) Inventor: DEBINSKI, Waldemar, Winston-Salem, NC 27104 (US); WYKOSKY, Jill, La Jolla, CA 92037 (US); GIBO, Denise, Winston-Salem, NC 27127 (US)
(74) Representative: Horner, Martin Grenville
(86) International application number: PCT/US2007/005964
(87) International publication number: WO 2007/103522

(56) References cited:
- WO-A-02/02799
- WO-A-03/004057
- WO-A-2005/042028
- WO-A-2006/047298
- WO-A2-2005/056766
- US-A1- 2004 180 823
- US-A1- 2005 147 593
- US-A1- 2005 249 736
- US-B1- 6 296 843
- BARTLEY T D ET AL: "B61 IS A LIGAND FOR THE ECK RECEPTOR PROTEIN-TYROSINE KINASE" NATURE, NATURE PUBLISHING GROUP, LONDON, UK, vol. 368, 7 April 1994 (1994-04-07), pages 558-560, XP001084298 ISSN: 0028-0836
- WYKOSKY J ET AL: "EPHA2 AS A NOVEL MOLECULAR MARKER AND TARGET IN GLIOBLASTOMA MULTIFORME" MOLECULAR CANCER RESEARCH, AMERICAN ASSOCIATION FOR CANCER RESEARCH (A AC R), US, vol. 3, no. 10, 1 October 2005 (2005-10-01), pages 541-551, XP008076096 ISSN: 1541-7786
- FLANAGAN J G ET AL: "The ephrins and Eph receptors in neural development" ANNUAL REVIEW OF NEUROSCIENCE 1998 US, vol. 21, 1998, pages 309-345, XP002526760 ISSN: 0147-006X
- WHITLOCK KELLI: "Glioma: Studies Offer New Drug Targets" ONCOLOGY TIMES, vol. 27, no. 18, 25 September 2005 (2005-09-25), pages 25-27, XP002526761
- RAZA ET AL.: 'Identification of necrosis-associated genes in glioblastoma by cDNA microarray analysis' CLINICAL CANCER RESEARCH vol. 10, January 2004, pages 212 - 221, XP008131026
- J. FRISEN: 'Ephrins and their Eph receptors: multitalented directors of embryonic development' THE EMBO JOURNAL vol. 18, no. 19, 01 October 1999, pages 5159 - 5165, XP055019467 DOI: 10.1093/emboj/18.19.5159

### SEQUENCE LISTING

<110> Wake Forest University Health Sciences
   Debinski, Waldemar
   Wykosky, Jill
   Gibo, Denise
<120> SOLUBLE MONOMERIC EPHRIN A1
<130> 9151-75WO
<150> US 60/780,241
   <151> 2006-03-08
<160> 16
<170> PatentIn version 3.3
<210> 1
   <211> 1519
   <212> DNA
   <213> Homo sapiens
<220>
   <221> CDS
   <222> (99)..(716)
<400> 1
   ccacgcgtcc ggcgctgact gcgccgcgga gaaagccagt gggaacccag acccatagga      60
   gacccgcgtc cccgctcggc ctggccaggc cccgcgct atg gag ttc ctc tgg gcc     116
   cct ctc ttg ggt ctg tgc tgc agt ctg gcc gct gct gat cgc cac acc      164
   gtc ttc tgg aac agt tca aat ccc aag ttc cgg aat gag gac tac acc      212
   ata cat gtg cag ctg aat gac tac gtg gac atc atc tgt ccg cac tat      260
   gaa gat cac tct gtg gca gac gct gcc atg gag cag tac ata ctg tac      308
   ctg gtg gag cat gag gag tac cag ctg tgc cag ccc cag tcc aag gac      356
   caa gtc cgc tgg cag tgc aac cgg ccc agt gcc aag cat ggc ccg gag      404
   aag ctg tct gag aag ttc cag cgc ttc aca cct ttc acc ctg ggc aag      452
   gag ttc aaa gaa gga cac agc tac tac tac atc tcc aaa ccc atc cac       500
   cag cat gaa gac cgc tgc ttg agg ttg aag gtg act gtc agt ggc aaa       548
   atc act cac agt cct cag gcc cat gtc aat cca cag gag aag aga ctt       596
   gca gca gat gac cca gag gtg cgg gtt cta cat agc atc gct cac agt       644
   gct gcc cca cgc ctc ttc cca ctt gcc tgg act gtg ctg ctc ctt cca       692
   ctt ctg ctg ctg caa acc ccg tga aggtgtatgc cacacctggc cttaaagagg      746
   gacaggctga agagagggac aggcactcca aacctgtctt ggggccactt tcagagcccc     806
   cagccctggg aaccactccc accacaggca taagctatca cctagcagcc tcaaaacggg     866
   tcagtattaa ggttttcaac cggaaggagg ccaaccagcc cgacagtgcc atccccacct     926
   tcacctcgga gggatggaga aagaagtgga gacagtcctt tcccaccatt cctgccttta     986
   agccaaagaa acaagctgtg caggcatggt cccttaaggc acagtgggag ctgagctgga    1046
   aggggccacg tggatgggca aagcttgtca aagatgcccc ctccaggaga gagccaggat    1106
   gcccagatga actgactgaa ggaaaagcaa gaaacagttt cttgcttgga agccaggtac    1166
   aggagaggca gcatgcttgg gctgacccag catctcccag caagacctca tctgtggagc    1226
   tgccacagag aagtttgtag ccaggtactg cattctctcc catcctgggg cagcactccc    1286
   cagagctgtg ccagcagggg ggctgtgcca acctgttctt agagtgtagc tgtaagggca    1346
   gtgcccatgt gtacattctg cctagagtgt agcctaaagg gcagggccca cgtgtatagt    1406
   atctgtatat aagttgctgt gtgtctgtcc tgatttctac aactggagtt tttttataca    1466
   atgttctttg tctcaaaata aagcaatgtg ttttttcgaa aaaaaaaaaa aaa              1519
<210> 2
   <211> 205
   <212> PRT
   <213> Homo sapiens
<400> 2
<210> 3
   <211> 1590
   <212> DNA
   <213> Homo sapiens
<220>
   <221> CDS
   <222> (106)..(723)
<400> 3
   gccagatctg tgagcccagc gctgactgcg ccgcggagaa agccagtggg aacccagacc      60
   cataggagac ccgcgtcccc gctcggcctg gccaggcccc gcgct atg gag ttc ctc     117
                                                     Met Glu Phe Leu
                                                     1
   tgg gcc cct ctc ttg ggt ctg tgc tgc agt ctg gcc gct gct gat cgc       165
   Trp Ala Pro Leu Leu Gly Leu Cys Cys Ser Leu Ala Ala Ala Asp Arg
   5                   10                  15                  20
   cac acc gtc ttc tgg aac agt tca aat ccc aag ttc cgg aat gag gac       213
   His Thr Val Phe Trp Asn Ser Ser Asn Pro Lys Phe Arg Asn Glu Asp
                   25                  30                  35
   tac acc ata cat gtg cag ctg aat gac tac gtg gac atc atc tgt ccg       261
   Tyr Thr Ile His Val Gln Leu Asn Asp Tyr Val Asp Ile Ile Cys Pro
               40                  45                  50
   cac tat gaa gat cac tct gtg gca gac gct gcc atg gag cag tac ata       309
   His Tyr Glu Asp His Ser Val Ala Asp Ala Ala Met Glu Gln Tyr Ile
           55                  60                  65
   ctg tac ctg gtg gag cat gag gag tac cag ctg tgc cag ccc cag tcc       357
   Leu Tyr Leu Val Glu His Glu Glu Tyr Gln Leu Cys Gln Pro Gln Ser
       70                  75                  80
   aag gac caa gtc cgc tgg cag tgc aac cgg ccc agt gcc aag cat ggc       405
   Lys Asp Gln Val Arg Trp Gln Cys Asn Arg Pro Ser Ala Lys His Gly
   85                  90                  95                  100
   ccg gag aag ctg tct gag aag ttc cag cgc ttc aca cct ttc acc ctg       453
   Pro Glu Lys Leu Ser Glu Lys Phe Gln Arg Phe Thr Pro Phe Thr Leu
                   105                 110                 115
   ggc aag gag ttc aaa gaa gga cac agc tac tac tac atc tcc aaa ccc       501
   Gly Lys Glu Phe Lys Glu Gly His Ser Tyr Tyr Tyr Ile Ser Lys Pro
               120                 125                 130
   atc cac cag cat gaa gac cgc tgc ttg agg ttg aag gtg act gtc agt       549
   Ile His Gln His Glu Asp Arg Cys Leu Arg Leu Lys Val Thr Val Ser
           135                 140                 145
   ggc aaa atc act cac agt cct cag gcc cat gac aat cca cag gag aag       597
   Gly Lys Ile Thr His Ser Pro Gln Ala His Asp Asn Pro Gln Glu Lys
       150                 155                 160
   aga ctt gca gca gat gac cca gag gtg cgg gtt cta cat agc atc ggt       645
   Arg Leu Ala Ala Asp Asp Pro Glu Val Arg Val Leu His Ser Ile Gly
   165                 170                 175                 180
   cac agt gct gcc cca cgc ctc ttc cca ctt gcc tgg act gtg ctg ctc       693
   His Ser Ala Ala Pro Arg Leu Phe Pro Leu Ala Trp Thr Val Leu Leu
                   185                 190                 195
   ctt cca ctt ctg ctg ctg caa acc ccg tga aggtgtatgc cacacctggc         743
   Leu Pro Leu Leu Leu Leu Gln Thr Pro
               200                 205
   cttaaagagg gacaggctga agagagggac aggcactcca aacctgtctt ggggccactt      803
   tcagagcccc cagccctggg aaccactccc accacaggca taagctatca cctagcagcc    863
   tcaaaacggg tcagtattaa ggttttcaac cggaaggagg ccaaccagcc cgacagtgcc    923
   atccccacct tcacctcgga gggatggaga aagaagtgga gacagtcctt tcccaccatt    983
   cctgccttta agccaaagaa acaagctgtg caggcatggt cccttaaggc acagtgggag   1043
   ctgagctgga aggggccacg tggatgggca aagcttgtca aagatgcccc ctccaggaga   1103
   gagccaggat gcccagatga actgactgaa ggaaaagcaa gaaacagttt cttgcttgga   1163
   agccaggtac aggagaggca gcatgcttgg gctgacccag catctcccag caagacctca   1223
   tctgtggagc tgccacagag aagtttgtag ccaggtactg cattctctcc catcctgggg   1283
   cagcactccc cagagctgtg ccagcagggg ggctgtgcca acctgttctt agagtgtagc   1343
   tgtaagggca gtgcccatgt gtacattctg cctagagtgt agcctaaagg gcagggccca   1403
   cgtgtatagt atctgtatat aagttgctgt gtgtctgtcc tgatttctac aactggagtt   1463
   tttttataca atgttctttg tctcaaaata aagcaatgtg ttttttcgga catgcttttc   1523
   tgccactcca tattaaaaca tatgaccatt gagtccctgc taaaaaaaaa aaaaaaaaaa   1583
   aaaaaaa                                                             1590
<210> 4
   <211> 205
   <212> PRT
   <213> Homo sapiens
<400> 4
   Met Glu Phe Leu Trp Ala Pro Leu Leu Gly Leu Cys Cys Ser Leu Ala
   1               5                   10                  15
   Ala Ala Asp Arg His Thr Val Phe Trp Asn Ser Ser Asn Pro Lys Phe
               20                  25                  30
   Arg Asn Glu Asp Tyr Thr Ile His Val Gln Leu Asn Asp Tyr Val Asp
           35                  40                  45
   Ile Ile Cys Pro His Tyr Glu Asp His Ser Val Ala Asp Ala Ala Met
       50                  55                  60
   Glu Gln Tyr Ile Leu Tyr Leu Val Glu His Glu Glu Tyr Gln Leu Cys
   65                  70                  75                  80
   Gln Pro Gln Ser Lys Asp Gln Val Arg Trp Gln Cys Asn Arg Pro Ser
                   85                  90                  95
   Ala Lys His Gly Pro Glu Lys Leu Ser Glu Lys Phe Gln Arg Phe Thr
   100 105 110
   Pro Phe Thr Leu Gly Lys Glu Phe Lys Glu Gly His Ser Tyr Tyr Tyr
   115 120 125
   Ile Ser Lys Pro Ile His Gln His Glu Asp Arg Cys Leu Arg Leu Lys
   130 135 140
   Val Thr Val Ser Gly Lys Ile Thr His Ser Pro Gln Ala His Asp Asn
   145 150 155 160
   Pro Gln Glu Lys Arg Leu Ala Ala Asp Asp Pro Glu Val Arg Val Leu
   165 170 175
   His Ser Ile Gly His Ser Ala Ala Pro Arg Leu Phe Pro Leu Ala Trp
   180 185 190
   Thr Val Leu Leu Leu Pro Leu Leu Leu Leu Gln Thr Pro
   195 200 205
<210> 5
   <211> 1524
   <212> DNA
   <213> Homo sapiens
<220>
   <221> CDS
   <222> (106).. (657)
<400> 5
   gccagatctg tgagcccagc gctgactgcg ccgcggagaa agccagtggg aacccagacc 60
   cataggagac ccgcgtcccc gctcggcctg gccaggcccc gcgct atg gag ttc ctc 117
   Met Glu Phe Leu
   1
   tgg gcc cct ctc ttg ggt ctg tgc tgc agt ctg gcc gct gct gat cgc 165
   Trp Ala Pro Leu Leu Gly Leu Cys Cys Ser Leu Ala Ala Ala Asp Arg
   5 10 15 20
   cac acc gtc ttc tgg aac agt tca aat ccc aag ttc cgg aat gag gac 213
   His Thr Val Phe Trp Asn Ser Ser Asn Pro Lys Phe Arg Asn Glu Asp
   25 30 35
   tac acc ata cat gtg cag ctg aat gac tac gtg gac atc atc tgt ccg 261
   Tyr Thr Ile His Val Gln Leu Asn Asp Tyr Val Asp Ile Ile Cys Pro
   40 45 50
   cac tat gaa gat cac tct gtg gca gac gct gcc atg gag cag tac ata 309
   His Tyr Glu Asp His Ser Val Ala Asp Ala Ala Met Glu Gln Tyr Ile
   55 60 65
   ctg tac ctg gtg gag cat gag gag tac cag ctg tgc cag ccc cag tcc 357
   Leu Tyr Leu Val Glu His Glu Glu Tyr Gln Leu Cys Gln Pro Gln Ser
       70                  75                  80
   aag gac caa gtc cgc tgg cag tgc aac cgg ccc agt gcc aag cat ggc      405
   Lys Asp Gln Val Arg Trp Gln Cys Asn Arg Pro Ser Ala Lys His Gly
   85                  90                  95                  100
   ccg gag aag ctg tct gag aag ttc cag cgc ttc aca cct ttc acc ctg      453
   Pro Glu Lys Leu Ser Glu Lys Phe Gln Arg Phe Thr Pro Phe Thr Leu
                   105                 110                 115
   ggc aag gag ttc aaa gaa gga cac agc tac tac tac atc tct cac agt      501
   Gly Lys Glu Phe Lys Glu Gly His Ser Tyr Tyr Tyr Ile Ser His Ser
               120                 125                 130
   cct cag gcc cat gac aat cca cag gag aag aga ctt gca gca gat gac      549
   Pro Gln Ala His Asp Asn Pro Gln Glu Lys Arg Leu Ala Ala Asp Asp
           135                 140                 145
   cca gag gtg cgg gtt cta cat agc atc ggt cac agt gct gcc cca cgc      597
   Pro Glu Val Arg Val Leu His Ser Ile Gly His Ser Ala Ala Pro Arg
       150                 155                 160
   ctc ttc cca ctt gcc tgg act gtg ctg ctc ctt cca ctt ctg ctg ctg      645
   Leu Phe Pro Leu Ala Trp Thr Val Leu Leu Leu Pro Leu Leu Leu Leu
   165                 170                 175                 180
   caa acc ccg tga aggtgtatgc cacacctggc cttaaagagg gacaggctga          697
   Gln Thr Pro
   agagagggac aggcactcca aacctgtctt ggggccactt tcagagcccc cagccctggg    757
   aaccactccc accacaggca taagctatca cctagcagcc tcaaaacggg tcagtattaa    817
   ggttttcaac cggaaggagg ccaaccagcc cgacagtgcc atccccacct tcacctcgga    877
   gggatggaga aagaagtgga gacagtcctt tcccaccatt cctgccttta agccaaagaa    937
   acaagctgtg caggcatggt cccttaaggc acagtgggag ctgagctgga aggggccacg    997
   tggatgggca aagcttgtca aagatgcccc ctccaggaga gagccaggat gcccagatga   1057
   actgactgaa ggaaaagcaa gaaacagttt cttgcttgga agccaggtac aggagaggca   1117
   gcatgcttgg gctgacccag catctcccag caagacctca tctgtggagc tgccacagag   1177
   aagtttgtag ccaggtactg cattctctcc catcctgggg cagcactccc cagagctgtg   1237
   ccagcagggg ggctgtgcca acctgttctt agagtgtagc tgtaagggca gtgcccatgt   1297
   gtacattctg cctagagtgt agcctaaagg gcagggccca cgtgtatagt atctgtatat   1357
   aagttgctgt gtgtctgtcc tgatttctac aactggagtt tttttataca atgttctttg   1417
   tctcaaaata aagcaatgtg ttttttcgga catgcttttc tgccactcca tattaaaaca   1477
   tatgaccatt gagtccctgc taaaaaaaaa aaaaaaaaaa aaaaaaa                 1524
<210> 6
   <211> 183
   <212> PRT
   <213> Homo sapiens
<400> 6
   Met Glu Phe Leu Trp Ala Pro Leu Leu Gly Leu Cys Cys Ser Leu Ala
   1               5                   10                  15
   Ala Ala Asp Arg His Thr Val Phe Trp Asn Ser Ser Asn Pro Lys Phe
               20                  25                  30
   Arg Asn Glu Asp Tyr Thr Ile His Val Gln Leu Asn Asp Tyr Val Asp
           35                  40                  45
   Ile Ile Cys Pro His Tyr Glu Asp His Ser Val Ala Asp Ala Ala Met
       50                  55                  60
   Glu Gln Tyr Ile Leu Tyr Leu Val Glu His Glu Glu Tyr Gln Leu Cys
   65                  70                  75                  80
   Gln Pro Gln Ser Lys Asp Gln Val Arg Trp Gln Cys Asn Arg Pro Ser
                   85                  90                  95
   Ala Lys His Gly Pro Glu Lys Leu Ser Glu Lys Phe Gln Arg Phe Thr
               100                 105                 110
   Pro Phe Thr Leu Gly Lys Glu Phe Lys Glu Gly His Ser Tyr Tyr Tyr
           115                 120                 125
   Ile Ser His Ser Pro Gln Ala His Asp Asn Pro Gln Glu Lys Arg Leu
       130                 135                 140
   Ala Ala Asp Asp Pro Glu Val Arg Val Leu His Ser Ile Gly His Ser
   145                 150                 155                 160
   Ala Ala Pro Arg Leu Phe Pro Leu Ala Trp Thr Val Leu Leu Leu Pro
                   165                 170                 175
   Leu Leu Leu Leu Gln Thr Pro
               180
<210> 7
   <211> 1474
   <212> DNA
   <213> Mus musculus
<220>
   <221> CDS
   <222> (101)..(718) .
<400> 7
   gagatttgtg atcgtggtgc ctactgtacc gtggagaagc ctgtgggaac cttacactgc      60
   agagctctcg tcctggcctg gctcggcccg gccccgcgct atg gag ttc ctt tgg       115
                                               Met Glu Phe Leu Trp
                                               1               5
   gcc cct ctc ttg ggt ctg tgc tgc agt ctg gcc gct gct gac cgc cac       163
   Ala Pro Leu Leu Gly Leu Cys Cys Ser Leu Ala Ala Ala Asp Arg His
                   10                  15                  20
   atc gtc ttc tgg aac agt tca aat ccc aag ttc cgt gag gag gac tac       211
   Ile Val Phe Trp Asn Ser Ser Asn Pro Lys Phe Arg Glu Glu Asp Tyr
               25                  30                  35
   acg gtg cac gtg cag ctg aat gac tac cta gac atc atc tgc cca cat       259
   Thr Val His Val Gln Leu Asn Asp Tyr Leu Asp Ile Ile Cys Pro His
           40                  45                  50
   tac gag gac gac tct gtg gca gat gca gcc atg gag cga tac aca ctg       307
   Tyr Glu Asp Asp Ser Val Ala Asp Ala Ala Met Glu Arg Tyr Thr Leu
       55                  60                  65
   tac atg gtg gaa cac cag gag tat gtg gca tgc caa ccc cag tcc aag       355
   Tyr Met Val Glu His Gln Glu Tyr Val Ala Cys Gln Pro Gln Ser Lys
   70                  75                  80                  85
   gac cag gtc cgt tgg aat tgc aac cgg ccc agt gcc aag cat ggc ccg       403
   Asp Gln Val Arg Trp Asn Cys Asn Arg Pro Ser Ala Lys His Gly Pro
                   90                  95                  100
   gag aag ctg tct gtg aaa ttc cag cgc ttc acg cct ttt atc ttg ggc       451
   Glu Lys Leu Ser Val Lys Phe Gln Arg Phe Thr Pro Phe Ile Leu Gly
               105                 110                 115
   aag gag ttc aag gaa gga cac agc tac tac tac atc tcc aaa cct atc       499
   Lys Glu Phe Lys Glu Gly His Ser Tyr Tyr Tyr Ile Ser Lys Pro Ile
           120                 125                 130
   tac cat cag gaa tcc cag tgc ttg aag ctg aag gtg act gtc aat ggc       547
   Tyr His Gln Glu Ser Gln Cys Leu Lys Leu Lys Val Thr Val Asn Gly
       135                 140                 145
   aaa atc act cat aat ccc cag gcc cat gtc aac cca cag gag aag aga       595
   Lys Ile Thr His Asn Pro Gln Ala His Val Asn Pro Gln Glu Lys Arg
   150                 155                 160                 165
   ctc caa gca gat gac ccg gaa gta cag gtt ttg cac agc att ggt tac       643
   Leu Gln Ala Asp Asp Pro Glu Val Gln Val Leu His Ser Ile Gly Tyr
                   170                 175                 180
   agt gcc gcc ccc cgc ctc ttc cca ctg gtc tgg gca gta ttg ctc cta       691
   Ser Ala Ala Pro Arg Leu Phe Pro Leu Val Trp Ala Val Leu Leu Leu
               185                 190                 195
   cca ctg ctg ctg ctg caa tct cag tga gggtgtacgc ttgccctggc             738
   Pro Leu Leu Leu Leu Gln Ser Gln
           200                 205
   ttatggattg gaatgggact aaggggcagc cccagccctg ggaacctctc ccactacacc    798
   cacaagacgc caccatgaag cctcaaaagg ttcagtatta agggttttaa ccgaaaagag    858
   tttaaccagc ccaactgtgc catccctgcc tccacttcag agggatggag aaagaagtgg    918
   agaaggtcct taacctgcag tttctgcctt taagccaaag aaacaagctg tgcggacctg    978
   gcccattaag aggcctcagt gggagaaggg ctaaaaagag cctgaggtca tgcggttgga   1038
   cactgaactt ggtaaagacg tccttcccca aggaagccat aatgtgcaga tgaactgacc   1098
   gaaggaaaag cttgagacag cttcctgcgg gggagccagg tacagaagag gcagcttggg   1158
   ctgacccagc atctctgcaa gatttccacc tgctgggctg ccagagaggt ttgtagcccg   1218
   gccctgactg catcctcccc atccctgggg caacattccc tggagctgtg ccaacaggag   1278
   gactgaagca gcctggtttt agagtgtagc tgtaagggca gtgcccgtgt gtacagtctg   1338
   tggagtttag cttaacgggc agggcccaca tgtacagtgt ctgtatataa atgtgctgta   1398
   tctgttcatt tctatgactg cagggttttt ttatacagtg tttttggatt cataataaat   1458
   tgatggtttt ttatgg                                                   1474
<210> 8
   <211> 205
   <212> PRT
   <213> Mus musculus
<400> 8
   Met Glu Phe Leu Trp Ala Pro Leu Leu Gly Leu Cys Cys Ser Leu Ala
   1               5                   10                  15
   Ala Ala Asp Arg His Ile Val Phe Trp Asn Ser Ser Asn Pro Lys Phe
               20                  25                  30
   Arg Glu Glu Asp Tyr Thr Val His Val Gln Leu Asn Asp Tyr Leu Asp
           35                  40                  45
   Ile Ile Cys Pro His Tyr Glu Asp Asp Ser Val Ala Asp Ala Ala Met
       50                  55                  60
   Glu Arg Tyr Thr Leu Tyr Met Val Glu His Gln Glu Tyr Val Ala Cys
   65                  70                  75                  80
   Gln Pro Gln Ser Lys Asp Gln Val Arg Trp Asn Cys Asn Arg Pro Ser
                   85                  90                  95
   Ala Lys His Gly Pro Glu Lys Leu Ser Val Lys Phe Gln Arg Phe Thr
               100                 105                 110
   Pro Phe Ile Leu Gly Lys Glu Phe Lys Glu Gly His Ser Tyr Tyr Tyr
   115 120 125
   Ile Ser Lys Pro Ile Tyr His Gln Glu Ser Gln Cys Leu Lys Leu Lys
   130 135 140
   Val Thr Val Asn Gly Lys Ile Thr His Asn Pro Gln Ala His Val Asn
   145 150 155 160
   Pro Gln Glu Lys Arg Leu Gln Ala Asp Asp Pro Glu Val Gln Val Leu
   165 170 175
   His Ser Ile Gly Tyr Ser Ala Ala Pro Arg Leu Phe Pro Leu Val Trp
   180 185 190
   Ala Val Leu Leu Leu Pro Leu Leu Leu Leu Gln Ser Gln
   195 200 205
<210> 9
   <211> 1740
   <212> DNA
   <213> Artificial
<220>
   <223> DT390-ephrin A1 fusion construct
<220>
   <221> CDS
   <222> (4)..(1737)
<220>
   <221> misc_feature
   <222> (987)..(987)
   <223> n is a, c, g, or t
<400> 9
   cat atg ggc gct gat gat gtt gtt gat tct tct aaa tct ttt gtg atg 48
   Met Gly Ala Asp Asp Val Val Asp Ser Ser Lys Ser Phe Val Met
   1 5 10 15
   gaa aac ttt tct tcg tac cac ggg act aaa cct ggt tat gta gat tcc 96
   Glu Asn Phe Ser Ser Tyr His Gly Thr Lys Pro Gly Tyr Val Asp Ser
   20 25 30
   att caa aaa ggt ata caa aag cca aaa tct ggt aca caa gga aat tat 144
   Ile Gln Lys Gly Ile Gln Lys Pro Lys Ser Gly Thr Gln Gly Asn Tyr
   35 40 45
   gac gat gat tgg aaa ggg ttt tat agt acc gac aat aaa tac gac gct 192
   Asp Asp Asp Trp Lys Gly Phe Tyr Ser Thr Asp Asn Lys Tyr Asp Ala
   50 55 60
   gcg gga tac tct gta gat aat gaa aac ccg ctc tct gga aaa gct gga      240
   Ala Gly Tyr Ser Val Asp Asn Glu Asn Pro Leu Ser Gly Lys Ala Gly
       65                  70                  75
   ggc gtg gtc aaa gtg acg tat cca gga ctg acg aag gtt ctc gca cta      288
   Gly Val Val Lys Val Thr Tyr Pro Gly Leu Thr Lys Val Leu Ala Leu
   80                  85                  90                  95
   aaa gtg gat aat gcc gaa act att aag aaa gag tta ggt tta agt ctc      336
   Lys Val Asp Asn Ala Glu Thr Ile Lys Lys Glu Leu Gly Leu Ser Leu
                   100                 105                 110
   act gaa ccg ttg atg gag caa gtc gga acg gaa gag ttt atc aaa agg      384
   Thr Glu Pro Leu Met Glu Gln Val Gly Thr Glu Glu Phe Ile Lys Arg
               115                 120                 125
   ttc ggt gat ggt gct tcg cgt gta gtg ctc agc ctt ccc ttc gct gag      432
   Phe Gly Asp Gly Ala Ser Arg Val Val Leu Ser Leu Pro Phe Ala Glu
           130                 135                 140
   ggg agt tct agc gtt gaa tat att aat aac tgg gaa cag gcg aaa gcg      480
   Gly Ser Ser Ser Val Glu Tyr Ile Asn Asn Trp Glu Gln Ala Lys Ala
       145                 150                 155
   tta agc gta gaa ctt gag att aat ttt gaa acc cgt gga aaa cgt ggc      528
   Leu Ser Val Glu Leu Glu Ile Asn Phe Glu Thr Arg Gly Lys Arg Gly
   160                 165                 170                 175
   caa gat gcg atg tat gag tat atg gct caa gcc tgt gca gga aat cgt      576
   Gln Asp Ala Met Tyr Glu Tyr Met Ala Gln Ala Cys Ala Gly Asn Arg
                   180                 185                 190
   gtc agg cga tca gta ggt agc tca ttg tca tgc ata aat ctt gat tgg      624
   Val Arg Arg Ser Val Gly Ser Ser Leu Ser Cys Ile Asn Leu Asp Trp
               195                 200                 205
   gat gtc ata agg gat aaa act aag aca aag ata gag tct ttg aaa gag      672
   Asp Val Ile Arg Asp Lys Thr Lys Thr Lys Ile Glu Ser Leu Lys Glu
           210                 215                 220
   cat ggc cct atc aaa aat aaa atg agc gaa agt ccc aat aaa aca gta      720
   His Gly Pro Ile Lys Asn Lys Met Ser Glu Ser Pro Asn Lys Thr Val
       225                 230                 235
   tct gag gaa aaa gct aaa caa tac cta gaa gaa ttt cat caa acg gca      768
   Ser Glu Glu Lys Ala Lys Gln Tyr Leu Glu Glu Phe His Gln Thr Ala
   240                 245                 250                 255
   tta gag cat cct gaa ttg tca gaa ctt aaa acc gtt act ggg acc aat      816
   Leu Glu His Pro Glu Leu Ser Glu Leu Lys Thr Val Thr Gly Thr Asn
                   260                 265                 270
   cct gta ttc gct ggg gct aac tat gcg gcg tgg gca gta aac gtt gcg      864
   Pro Val Phe Ala Gly Ala Asn Tyr Ala Ala Trp Ala Val Asn Val Ala
               275                 280                 285
   caa gtt atc gat agc gaa aca gct gat aat ttg gaa aag aca act gct      912
   Gln Val Ile Asp Ser Glu Thr Ala Asp Asn Leu Glu Lys Thr Thr Ala
           290                 295                 300
   gct ctt tcg ata ctt cct ggt atc ggt agc gta atg ggc att gca gac      960
   Ala Leu Ser Ile Leu Pro Gly Ile Gly Ser Val Met Gly Ile Ala Asp
       305                 310                 315
   ggt gcc gtt cac cac aat aca gaa gan ata gtg gcg caa tca ata gct      1008
   Gly Ala Val His His Asn Thr Glu Xaa Ile Val Ala Gln Ser Ile Ala
   320                 325                 330                 335
   tta tcg tct tta atg gtt gct caa gct att cca ttg gta gga gag cta      1056
   Leu Ser Ser Leu Met Val Ala Gln Ala Ile Pro Leu Val Gly Glu Leu
                   340                 345                 350
   gtt gat att ggt ttc gct gca tat aat ttt gta gag agt att atc aat      1104
   Val Asp Ile Gly Phe Ala Ala Tyr Asn Phe Val Glu Ser Ile Ile Asn
               355                 360                 365
   tta ttt caa gta gtt cat aat tcg tat aat cgt ccc gcg tat tct ccg      1152
   Leu Phe Gln Val Val His Asn Ser Tyr Asn Arg Pro Ala Tyr Ser Pro
           370                 375                 380
   ggg cat aaa acg caa cca aag ctt gat cgc cac acc gtc ttc tgg aac      1200
   Gly His Lys Thr Gln Pro Lys Leu Asp Arg His Thr Val Phe Trp Asn
       385                 390                 395
   agt tca aat ccc aag ttc cgg aat gag gac tac acc ata cat gtg cag      1248
   Ser Ser Asn Pro Lys Phe Arg Asn Glu Asp Tyr Thr Ile His Val Gln
   400                 405                 410                 415
   ctg aat gac tac gtg gac atc atc tgt ccg cac tat gaa gat cac tct      1296
   Leu Asn Asp Tyr Val Asp Ile Ile Cys Pro His Tyr Glu Asp His Ser
                   420                 425                 430
   gtg gca gac gct gcc atg gag cag tac ata ctg tac ctg gtg gag cat      1344
   Val Ala Asp Ala Ala Met Glu Gln Tyr Ile Leu Tyr Leu Val Glu His
               435                 440                 445
   gag gag tac cag ctg tgc cag ccc cag tcc aag gac caa gtc cgc tgg      1392
   Glu Glu Tyr Gln Leu Cys Gln Pro Gln Ser Lys Asp Gln Val Arg Trp
           450                 455                 460
   cag tgc aac cgg ccc agt gcc aag cat ggc ccg gag aag ctg tct gag      1440
   Gln Cys Asn Arg Pro Ser Ala Lys His Gly Pro Glu Lys Leu Ser Glu
       465                 470                 475
   aag ttc cag cgc ttc aca cct ttc acc ctg ggc aag gag ttc aaa gaa      1488
   Lys Phe Gln Arg Phe Thr Pro Phe Thr Leu Gly Lys Glu Phe Lys Glu
   480                 485                 490                 495
   gga cac agc tac tac tac atc tcc aaa ccc atc cac cag cat gaa gac      1536
   Gly His Ser Tyr Tyr Tyr Ile Ser Lys Pro Ile His Gln His Glu Asp
                   500                 505                 510
   cgc tgc ttg agg ttg aag gtg act gtc agt ggc aaa atc act cac agt      1584
   Arg Cys Leu Arg Leu Lys Val Thr Val Ser Gly Lys Ile Thr His Ser
               515                 520                 525
   cct cag gcc cat gtc aat cca cag gag aag aga ctt gca gca gat gac      1632
   Pro Gln Ala His Val Asn Pro Gln Glu Lys Arg Leu Ala Ala Asp Asp
           530                 535                 540
   cca gag gtg cgg gtt cta cat agc atc gct cac agt gct gcc cca cgc      1680
   Pro Glu Val Arg Val Leu His Ser Ile Ala His Ser Ala Ala Pro Arg
   545 550 555
   ctc ttc cca ctt gcc tgg act gtg ctg ctc ctt cca ctt ctg ctg ctg 1728
   Leu Phe Pro Leu Ala Trp Thr Val Leu Leu Leu Pro Leu Leu Leu Leu
   560 565 570 575
   caa acc ccg tga 1740
   Gln Thr Pro
<210> 10
   <211> 578
   <212> PRT
   <213> Artificial
<220>
   <221> misc_feature
   <222> (328)..(328)
   <223> The 'Xaa' at location 328 stands for Glu, or Asp.
<220>
   <223> Synthetic Construct
<400> 10
   Met Gly Ala Asp Asp Val Val Asp Ser Ser Lys Ser Phe Val Met Glu
   1 5 10 15
   Asn Phe Ser Ser Tyr His Gly Thr Lys Pro Gly Tyr Val Asp Ser Ile
   20 25 30
   Gln Lys Gly Ile Gln Lys Pro Lys Ser Gly Thr Gln Gly Asn Tyr Asp
   35 40 45
   Asp Asp Trp Lys Gly Phe Tyr Ser Thr Asp Asn Lys Tyr Asp Ala Ala
   50 55 60
   Gly Tyr Ser Val Asp Asn Glu Asn Pro Leu Ser Gly Lys Ala Gly Gly
   65 70 75 80
   Val Val Lys Val Thr Tyr Pro Gly Leu Thr Lys Val Leu Ala Leu Lys
   85 90 95
   Val Asp Asn Ala Glu Thr Ile Lys Lys Glu Leu Gly Leu Ser Leu Thr
   100 105 110
   Glu Pro Leu Met Glu Gln Val Gly Thr Glu Glu Phe Ile Lys Arg Phe
   115 120 125
   Gly Asp Gly Ala Ser Arg Val Val Leu Ser Leu Pro Phe Ala Glu Gly
   130 135 140
   Ser Ser Ser Val Glu Tyr Ile Asn Asn Trp Glu Gln Ala Lys Ala Leu
   145                 150                 155                 160
   Ser Val Glu Leu Glu Ile Asn Phe Glu Thr Arg Gly Lys Arg Gly Gln
                   165                 170                 175
   Asp Ala Met Tyr Glu Tyr Met Ala Gln Ala Cys Ala Gly Asn Arg Val
               180                 185                 190
   Arg Arg Ser Val Gly Ser Ser Leu Ser Cys Ile Asn Leu Asp Trp Asp
           195                 200                 205
   Val Ile Arg Asp Lys Thr Lys Thr Lys Ile Glu Ser Leu Lys Glu His
       210                 215                 220
   Gly Pro Ile Lys Asn Lys Met Ser Glu Ser Pro Asn Lys Thr Val Ser
   225                 230                 235                 240
   Glu Glu Lys Ala Lys Gln Tyr Leu Glu Glu Phe His Gln Thr Ala Leu
                   245                 250                 255
   Glu His Pro Glu Leu Ser Glu Leu Lys Thr Val Thr Gly Thr Asn Pro
               260                 265                 270
   Val Phe Ala Gly Ala Asn Tyr Ala Ala Trp Ala Val Asn Val Ala Gln
           275                 280                 285
   Val Ile Asp Ser Glu Thr Ala Asp Asn Leu Glu Lys Thr Thr Ala Ala
       290                 295                 300
   Leu Ser Ile Leu Pro Gly Ile Gly Ser Val Met Gly Ile Ala Asp Gly
   305                 310                 315                 320
   Ala Val His His Asn Thr Glu Xaa Ile Val Ala Gln Ser Ile Ala Leu
                   325                 330                 335
   Ser Ser Leu Met Val Ala Gln Ala Ile Pro Leu Val Gly Glu Leu Val
               340                 345                 350
   Asp Ile Gly Phe Ala Ala Tyr Asn Phe Val Glu Ser Ile Ile Asn Leu
           355                 360                 365
   Phe Gln Val Val His Asn Ser Tyr Asn Arg Pro Ala Tyr Ser Pro Gly
       370                 375                 380
   His Lys Thr Gln Pro Lys Leu Asp Arg His Thr Val Phe Trp Asn Ser
   385 390 395 400
   Ser Asn Pro Lys Phe Arg Asn Glu Asp Tyr Thr Ile His Val Gln Leu
   405 410 415
   Asn Asp Tyr Val Asp Ile Ile Cys Pro His Tyr Glu Asp His Ser Val
   420 425 430
   Ala Asp Ala Ala Met Glu Gln Tyr Ile Leu Tyr Leu Val Glu His Glu
   435 440 445
   Glu Tyr Gln Leu Cys Gln Pro Gln Ser Lys Asp Gln Val Arg Trp Gln
   450 455 460
   Cys Asn Arg Pro Ser Ala Lys His Gly Pro Glu Lys Leu Ser Glu Lys
   465 470 475 480
   Phe Gln Arg Phe Thr Pro Phe Thr Leu Gly Lys Glu Phe Lys Glu Gly
   485 490 495
   His Ser Tyr Tyr Tyr Ile Ser Lys Pro Ile His Gln His Glu Asp Arg
   500 505 510
   Cys Leu Arg Leu Lys Val Thr Val Ser Gly Lys Ile Thr His Ser Pro
   515 520 525
   Gln Ala His Val Asn Pro Gln Glu Lys Arg Leu Ala Ala Asp Asp Pro
   530 535 540
   Glu Val Arg Val Leu His Ser Ile Ala His Ser Ala Ala Pro Arg Leu
   545 550 555 560
   Phe Pro Leu Ala Trp Thr Val Leu Leu Leu Pro Leu Leu Leu Leu Gln
   565 570 575
   Thr Pro
<210> 11
   <211> 1626
   <212> DNA
   <213> Artificial
<220>
   <223> Ephrin A1-PE38QQR fusion construct
<220>
   <221> CDS
   <222> (7) .. (1626)
<400> 11
   aagctt atg gat cgc cac acc gtc ttc tgg aac agt tca aat ccc aag      48
          Met Asp Arg His Thr Val Phe Trp Asn Ser Ser Asn Pro Lys
          1               5                   10
   ttc cgg aat gag gac tac acc ata cat gtg cag ctg aat gac tac gtg     96
   Phe Arg Asn Glu Asp Tyr Thr Ile His Val Gln Leu Asn Asp Tyr Val
   15                  20                  25                  30
   gac atc atc tgt ccg cac tat gaa gat cac tct gtg gca gac gct gcc    144
   Asp Ile Ile Cys Pro His Tyr Glu Asp His Ser Val Ala Asp Ala Ala
                   35                  40                  45
   atg gag cag tac ata ctg tac ctg gtg gag cat gag gag tac cag ctg    192
   Met Glu Gln Tyr Ile Leu Tyr Leu Val Glu His Glu Glu Tyr Gln Leu
               50                  55                  60
   tgc cag ccc cag tcc aag gac caa gtc cgc tgg cag tgc aac cgg ccc    240
   Cys Gln Pro Gln Ser Lys Asp Gln Val Arg Trp Gln Cys Asn Arg Pro
           65                  70                  75
   agt gcc aag cat ggc ccg gag aag ctg tct gag aag ttc cag cgc ttc    288
   Ser Ala Lys His Gly Pro Glu Lys Leu Ser Glu Lys Phe Gln Arg Phe
       80                  85                  90
   aca cct ttc acc ctg ggc aag gag ttc aaa gaa gga cac agc tac tac    336
   Thr Pro Phe Thr Leu Gly Lys Glu Phe Lys Glu Gly His Ser Tyr Tyr
   95                  100                 105                 110
   tac atc tcc aaa ccc atc cac cag cat gaa gac cgc tgc ttg agg ttg    384
   Tyr Ile Ser Lys Pro Ile His Gln His Glu Asp Arg Cys Leu Arg Leu
                   115                 120                 125
   aag gtg act gtc agt ggc aaa atc act cac agt cct cag gcc cat gtc    432
   Lys Val Thr Val Ser Gly Lys Ile Thr His Ser Pro Gln Ala His Val
               130                 135                 140
   aat cca cag gag aag aga ctt gca gca gat gac cca gag gtg cgg gtt    480
   Asn Pro Gln Glu Lys Arg Leu Ala Ala Asp Asp Pro Glu Val Arg Val
           145                 150                 155
   cta cat agc atc gct cac agt gct gcc cca cgc ctc ttc cca ctt gcc    528
   Leu His Ser Ile Ala His Ser Ala Ala Pro Arg Leu Phe Pro Leu Ala
       160                 165                 170
   tgg act gtg ctg ctc ctt cca ctt ctg ctg ctg caa acc ccg aga att    576
   Trp Thr Val Leu Leu Leu Pro Leu Leu Leu Leu Gln Thr Pro Arg Ile
   175                 180                 185                 190
   ccc gga ggt ccc gag ggc ggc agc ctg gcc gcg ctg acc gcg cac cag    624
   Pro Gly Gly Pro Glu Gly Gly Ser Leu Ala Ala Leu Thr Ala His Gln
                   195                 200                 205
   gct tgc cac ctg ccg ctg gag act ttc acc cgt cat cgc cag ccg cgc    672
   Ala Cys His Leu Pro Leu Glu Thr Phe Thr Arg His Arg Gln Pro Arg
               210                 215                 220
   ggc tgg gaa caa ctg gag cag tgc ggc tat ccg gtg cag cgg ctg gtc      720
   Gly Trp Glu Gln Leu Glu Gln Cys Gly Tyr Pro Val Gln Arg Leu Val
           225                 230                 235
   gcc ctc tac ctg gcg gcg cgg ctg tcg tgg aac cag gtc gac cag gtg      768
   Ala Leu Tyr Leu Ala Ala Arg Leu Ser Trp Asn Gln Val Asp Gln Val
       240                 245                 250
   atc cgc aac gcc ctg gcc agc ccc ggc agc ggc ggc gac ctg ggc gaa      816
   Ile Arg Asn Ala Leu Ala Ser Pro Gly Ser Gly Gly Asp Leu Gly Glu
   255                 260                 265                 270
   gcg atc cgc gag cag ccg gag caa gcc cgt ctg gcc ctg acc ctg gcc      864
   Ala Ile Arg Glu Gln Pro Glu Gln Ala Arg Leu Ala Leu Thr Leu Ala
                   275                 280                 285
   gcc gcc gag agc gag cgc ttc gtc cgg cag ggc acc ggc aac gac gag      912
   Ala Ala Glu Ser Glu Arg Phe Val Arg Gln Gly Thr Gly Asn Asp Glu
               290                 295                 300
   gcc ggc gcg gcc aac ggc ccg gcg gac agc ggc gac gcc ctg ctg gag      960
   Ala Gly Ala Ala Asn Gly Pro Ala Asp Ser Gly Asp Ala Leu Leu Glu
           305                 310                 315
   cgc aac tat ccc act ggc gcg gag ttc ctc ggc gac ggc ggc gac gtc      1008
   Arg Asn Tyr Pro Thr Gly Ala Glu Phe Leu Gly Asp Gly Gly Asp Val
       320                 325                 330
   agc ttc agc acc cgc ggc acg cag aac tgg acg gtg gag cgg ctg ctc      1056
   Ser Phe Ser Thr Arg Gly Thr Gln Asn Trp Thr Val Glu Arg Leu Leu
   335                 340                 345                 350
   cag gcg cac cgc caa ctg gag gag cgc ggc tat gtg ttc gtc ggc tac      1104
   Gln Ala His Arg Gln Leu Glu Glu Arg Gly Tyr Val Phe Val Gly Tyr
                   355                 360                 365
   cac ggc acc ttc ctc gaa gcg gcg caa agc atc gtc ttc ggc ggg gtg      1152
   His Gly Thr Phe Leu Glu Ala Ala Gln Ser Ile Val Phe Gly Gly Val
               370                 375                 380
   cgc gcg cgc agc cag gac ctc gac gcg atc tgg cgc ggt ttc tat atc      1200
   Arg Ala Arg Ser Gln Asp Leu Asp Ala Ile Trp Arg Gly Phe Tyr Ile
           385                 390                 395
   gcc ggc gat ccg gcg ctg gcc tac ggc tac gcc cag gac cag gaa ccc      1248
   Ala Gly Asp Pro Ala Leu Ala Tyr Gly Tyr Ala Gln Asp Gln Glu Pro
       400                 405                 410
   gac gca cgc ggc cgg atc cgc aac ggt gcc ctg ctg cgg gtc tat gtg      1296
   Asp Ala Arg Gly Arg Ile Arg Asn Gly Ala Leu Leu Arg Val Tyr Val
   415                 420                 425                 430
   ccg cgc tcg agc ctg ccg ggc ttc tac cgc acc agc ctg acc ctg gcc      1344
   Pro Arg Ser Ser Leu Pro Gly Phe Tyr Arg Thr Ser Leu Thr Leu Ala
                   435                 440                 445
   gcg ccg gag gcg gcg ggc gag gtc gaa cgg ctg atc ggc cat ccg ctg      1392
   Ala Pro Glu Ala Ala Gly Glu Val Glu Arg Leu Ile Gly His Pro Leu
               450                 455                 460
   ccg ctg cgc ctg gac gcc atc acc ggc ccc gag gag gaa ggc ggg cgc      1440
   Pro Leu Arg Leu Asp Ala Ile Thr Gly Pro Glu Glu Glu Gly Gly Arg
   465 470 475
   ctg gag acc att ctc ggc tgg ccg ctg gcc gag cgc acc gtg gtg att 1488
   Leu Glu Thr Ile Leu Gly Trp Pro Leu Ala Glu Arg Thr Val Val Ile
   480 485 490
   ccc tcg gcg atc ccc acc gac ccg cgc aac gtc ggc ggc gac ctc gac 1536
   Pro Ser Ala Ile Pro Thr Asp Pro Arg Asn Val Gly Gly Asp Leu Asp
   495 500 505 510
   ccg tcc agc atc ccg gac caa gaa cag gcg atc agc gcc ctg ccg gac 1584
   Pro Ser Ser Ile Pro Asp Gln Glu Gln Ala Ile Ser Ala Leu Pro Asp
   515 520 525
   tac gcc agc cag ccc ggg caa ccg ccg cgc gag gac ctg cgt 1626
   Tyr Ala Ser Gln Pro Gly Gln Pro Pro Arg Glu Asp Leu Arg
   530 535 540
<210> 12
   <211> 540
   <212> PRT
   <213> Artificial
<220>
   <223> Synthetic Construct
<400> 12
   Met Asp Arg His Thr Val Phe Trp Asn Ser Ser Asn Pro Lys Phe Arg
   1 5 10 15
   Asn Glu Asp Tyr Thr Ile His Val Gln Leu Asn Asp Tyr Val Asp Ile
   20 25 30
   Ile Cys Pro His Tyr Glu Asp His Ser Val Ala Asp Ala Ala Met Glu
   35 40 45
   Gln Tyr Ile Leu Tyr Leu Val Glu His Glu Glu Tyr Gln Leu Cys Gln
   50 55 60
   Pro Gln Ser Lys Asp Gln Val Arg Trp Gln Cys Asn Arg Pro Ser Ala
   65 70 75 80
   Lys His Gly Pro Glu Lys Leu Ser Glu Lys Phe Gln Arg Phe Thr Pro
   85 90 95
   Phe Thr Leu Gly Lys Glu Phe Lys Glu Gly His Ser Tyr Tyr Tyr Ile
   100 105 110
   Ser Lys Pro Ile His Gln His Glu Asp Arg Cys Leu Arg Leu Lys Val
   115 120 125
   Thr Val Ser Gly Lys Ile Thr His Ser Pro Gln Ala His Val Asn Pro
   130 135 140
   Gln Glu Lys Arg Leu Ala Ala Asp Asp Pro Glu Val Arg Val Leu His
   145 150 155 160
   Ser Ile Ala His Ser Ala Ala Pro Arg Leu Phe Pro Leu Ala Trp Thr
   165 170 175
   Val Leu Leu Leu Pro Leu Leu Leu Leu Gln Thr Pro Arg Ile Pro Gly
   180 185 190
   Gly Pro Glu Gly Gly Ser Leu Ala Ala Leu Thr Ala His Gln Ala Cys
   195 200 205
   His Leu Pro Leu Glu Thr Phe Thr Arg His Arg Gln Pro Arg Gly Trp
   210 215 220
   Glu Gln Leu Glu Gln Cys Gly Tyr Pro Val Gln Arg Leu Val Ala Leu
   225 230 235 240
   Tyr Leu Ala Ala Arg Leu Ser Trp Asn Gln Val Asp Gln Val Ile Arg
   245 250 255
   Asn Ala Leu Ala Ser Pro Gly Ser Gly Gly Asp Leu Gly Glu Ala Ile
   260 265 270
   Arg Glu Gln Pro Glu Gln Ala Arg Leu Ala Leu Thr Leu Ala Ala Ala
   275 280 285
   Glu Ser Glu Arg Phe Val Arg Gln Gly Thr Gly Asn Asp Glu Ala Gly
   290 295 300
   Ala Ala Asn Gly Pro Ala Asp Ser Gly Asp Ala Leu Leu Glu Arg Asn
   305 310 315 320
   Tyr Pro Thr Gly Ala Glu Phe Leu Gly Asp Gly Gly Asp Val Ser Phe
   325 330 335
   Ser Thr Arg Gly Thr Gln Asn Trp Thr Val Glu Arg Leu Leu Gln Ala
   340 345 350
   His Arg Gln Leu Glu Glu Arg Gly Tyr Val Phe Val Gly Tyr His Gly
   355 360 365
   Thr Phe Leu Glu Ala Ala Gln Ser Ile Val Phe Gly Gly Val Arg Ala
       370                 375                 380
   Arg Ser Gln Asp Leu Asp Ala Ile Trp Arg Gly Phe Tyr Ile Ala Gly
   385                 390                 395                 400
   Asp Pro Ala Leu Ala Tyr Gly Tyr Ala Gln Asp Gln Glu Pro Asp Ala
                   405                 410                 415
   Arg Gly Arg Ile Arg Asn Gly Ala Leu Leu Arg Val Tyr Val Pro Arg
               420                 425                 430
   Ser Ser Leu Pro Gly Phe Tyr Arg Thr Ser Leu Thr Leu Ala Ala Pro
           435                 440                 445
   Glu Ala Ala Gly Glu Val Glu Arg Leu Ile Gly His Pro Leu Pro Leu
       450                 455                 460
   Arg Leu Asp Ala Ile Thr Gly Pro Glu Glu Glu Gly Gly Arg Leu Glu
   465                 470                 475                 480
   Thr Ile Leu Gly Trp Pro Leu Ala Glu Arg Thr Val Val Ile Pro Ser
                   485                 490                 495
   Ala Ile Pro Thr Asp Pro Arg Asn Val Gly Gly Asp Leu Asp Pro Ser
               500                 505                 510
   Ser Ile Pro Asp Gln Glu Gln Ala Ile Ser Ala Leu Pro Asp Tyr Ala
           515                 520                 525
   Ser Gln Pro Gly Gln Pro Pro Arg Glu Asp Leu Arg
       530                 535                 540
<210> 13
   <211> 2406
   <212> DNA
   <213> Artificial
<220>
   <223> Ephrin A1-PE4E fusion construct
<220>
   <221> CDS
   <222> (7)..(2403)
<400> 13
   aagctt atg gat cgc cac acc gtc ttc tgg aac agt tca aat ccc aag      48
   ttc cgg aat gag gac tac acc ata cat gtg cag ctg aat gac tac gtg      96
   gac atc atc tgt ccg cac tat gaa gat cac tct gtg gca gac gct gcc     144
   atg gag cag tac ata ctg tac ctg gtg gag cat gag gag tac cag ctg     192
   tgc cag ccc cag tcc aag gac caa gtc cgc tgg cag tgc aac cgg ccc     240
   agt gcc aag cat ggc ccg gag aag ctg tct gag aag ttc cag cgc ttc     288
   aca cct ttc acc ctg ggc aag gag ttc aaa gaa gga cac agc tac tac     336
   tac atc tcc aaa ccc atc cac cag cat gaa gac cgc tgc ttg agg ttg     384
   aag gtg act gtc agt ggc aaa atc act cac agt cct cag gcc cat gtc     432
   aat cca cag gag aag aga ctt gca gca gat gac cca gag gtg cgg gtt     480
   cta cat agc atc gct cac agt gct gcc cca cgc ctc ttc cca ctt gcc     528
   tgg act gtg ctg ctc ctt cca ctt ctg ctg ctg caa acc ccg aaa gct     576
   ttc gac ctc tgg aac gaa tgc gcc aaa gcc tgc gtg ctc gac ctc aag     624
   gac ggc gtg cgt tcc agc cgc atg agc gtc gac ccg gcc atc gcc gac     672
   acc aac ggc cag ggc gtg ctg cac tac tcc atg gtc ctg gag ggc ggc     720
   aac gac gcg ctc gag ctg gcc atc gac aac gcc ctc agc atc acc agc     768
   gac ggc ctg acc atc cgc ctc gaa ggc ggc gtc gag ccg aac aag ccg      816
   gtg cgc tac agc tac acg cgc cag gcg cgc ggc agt tgg tcg ctg aac      864
   tgg ctg gta ccg atc ggc cac gag aag ccc tcg aac atc aag gtg ttc      912
   atc cac gaa ctg aac gcc ggc aac cag ctc agc cac atg tcg ccg atc      960
   tac acc atc gag atg ggc gac gag ttg ctg gcg aag ctg gcg cgc gat     1008
   gcc acc ttc ttc gtc agg gcg cac gag agc aac gag atg cag ccg acg     1056
   ctc gcc atc agc cat gcc ggg gtc agc gtg gtc atg gcc cag acc cag     1104
   ccg cgc cgg gaa aag cgc tgg agc gaa tgg gcc agc ggc aag gtg ttg     1152
   tgc ctg ctc gac ccg ctg gac ggg gtc tac aac tac ctc gcc cag caa     1200
   cgc tgc aac ctc gac gat acc tgg gaa ggc aag atc tac cgg gtg ctc     1248
   gcc ggc aac ccg gcg aag cat gac ctg gac atc aaa ccc acg gtc atc     1296
   agt gaa gaa ctg gaa ttt cct gag ggc ggc agc ctg gcc gcg ctg acc     1344
   gcg cac cag gct tgc cac ctg ccg ctg gag act ttc acc cgt cat cgc     1392
   cag ccg cgc ggc tgg gaa caa ctg gag cag tgc ggc tat ccg gtg cag     1440
   cgg ctg gtc gcc ctc tac ctg gcg gcg cgg ctg tcg tgg aac cag gtc     1488
   gac cag gtg atc cgc aac gcc ctg gcc agc ccc ggc agc ggc ggc gac     1536
   ctg ggc gaa gcg atc cgc gag cag ccg gag cag gcc cgt ctg gcc ctg      1584
   acc ctg gcc gcc gcc gag agc gag cgc ttc gtc cgg cag ggc acc ggc      1632
   aac gac gag gcc ggc gcg gcc aac gcc gac gtg gtg agc ctg acc tgc      1680
   ccg gtc gcc gcc ggt gaa tgc gcg ggc ccg gcg gac agc ggc gac gcc      1728
   ctg ctg gag cgc aac tat ccc act ggc gcg gag ttc ctc ggc gac ggc      1776
   ggc gac gtc agc ttc agc acc cgc ggc acg cag aac tgg acg gtg gag      1824
   cgg ctg ctc cag gcg cac cgc caa ctg gag gag cgc ggc tat gtg ttc      1872
   gtc ggc tac cac ggc acc ttc ctc gaa gcg gcg caa agc atc gtc ttc      1920
   ggc ggg gtg cgc gcg cgc agc cag gac ctc gac gcg atc tgg cgc ggt      1968
   ttc tat atc gcc ggc gat ccg gcg ctg gcc tac ggc tac gcc cag gac      2016
   cag gaa ccc gac gca cgc ggc cgg atc cgc aac ggt gcc ctg ctg cgg      2064
   gtc tat gtg ccg cgc tcg agc ctg ccg ggc ttc tac cgc acc agc ctg      2112
   acc ctg gcc gcg ccg gag gcg gcg ggc gag gtc gaa cgg ctg atc ggc      2160
   cat ccg ctg ccg ctg cgc ctg gac gcc atc acc ggc ccc gag gag gaa      2208
   ggc ggg cgc ctg gag acc att ctc ggc tgg ccg ctg gcc gag cgc acc      2256
   gtg gtg att ccc tcg gcg atc ccc acc gac ccg cgc aac gtc ggc ggc      2304
   gac ctc gac ccg tcc agc atc ccc gac aag gaa cag gcg atc agc gcc      2352
   ctg ccg gac tac gcc agc cag ccc ggc aaa ccg ccg cgc gag gac ctg      2400
   aag taa                                                              2406
<210> 14
   <211> 799
   <212> PRT
   <213> Artificial
<220>
   <223> Synthetic Construct
<400> 14
<210> 15
   <211> 19
   <212> RNA
   <213> Artificial
<220>
   <223> siRNA sequence
<400> 15
   ugaaugacau gccgaucua 19
<210> 16
   <211> 19
   <212> RNA
   <213> Artificial
<220>
   <223> siRNA sequence
<400> 16
   uagaucggca ugucauuca 19

## Description

### Field of the Invention

The present invention concerns compounds and compositions, for use in treating cancers such as glioblastoma multiforme.

### Background of the Invention

Cancer is now the number one cause of death in North America. Malignant tumors of the central nervous system (CNS) are the third leading cause of cancer-related deaths in adolescents and adults between the ages of 15 and 34, and in children, brain tumors are the leading cause of cancer death. Furthermore, the two-year survival rate for patients with glioblastoma multiforme (GBM), a high-grade glioma (HGG), grade IV, is less than 20% (Davis et al. (1998) J. Neurosurg. 88:1-10), and there has been a steady increase in the incidence of brain cancers during the last 20 years ("Reports from the front" (1995) Science 267:1414). Almost any cancer can metastasize to the CNS (Olson et al. (1974) Arch Neurol 30:122-136).

A common approach to the treatment of malignant gliomas involves surgery (Berger (1994) Sem Oncol 21:172-185), radiation therapy (Gunderson & Tepper, Eds. (2000) Clinical Radiation Oncology (Churchill-Livingstone, Philadelphia), pp 314-35), and various chemotherapeutic regimens (Lesser & Grossman (1994) Sem Oncol 21, 220-235), but neither single nor multimodal treatments are curative. At present, treatment is implemented to improve or sustain neurological function of the patient, to diminish the size of the tumor growing intracranially, and to lengthen intervals between treatments. Thus, new and molecularly specific methods of HGG treatment are urgently needed.

The transmembrane protein EphA2 is overexpressed and an attractive molecular target in glioblastoma multiforme (GBM), the most common primary brain tumor, which has a high incidence of recurrence and dim prognosis (Wykosky et al. (2005) Mol. Cancer Res. 3:541-551). Only 3% of patients survive five years, with a median survival of approximately14 months (Davis et al. (1998) J. Neurosurg. 88:1-10; Stupp et al. (2005) N. Engl. J. Med. 352:987-996). Interestingly, ephrinA1, a ligand for EphA2, is virtually not detectable in GBM cell lines, and is present at low levels in the majority of specimens despite the abundant overexpression of the receptor (Wykosky et al. (2005) Mol. Cancer Res. 3:541-551). A similar pattern of differential EphA2/ephrinA1 expression has been reported in breast cancer (Macrae et al. (2005) Cancer Cell 8:111-118).

The ephrins comprise a family of protein ligands for the Eph receptor tyrosine kinases, and are unique among ligands for receptor tyrosine kinases in that they have been described as GPI-linked (ephrinA) or transmembrane (ephrinB) cell surface proteins rather than soluble factors (Davis et al. (1994) Science 266:816-819). Hence, it has been widely accepted that endogenous activation of Eph receptors by their ephrin ligands requires stable cell-cell contact and/or clustering by ephrin ligands (Shao et al. (1995) J. Biol. Chem. 270:5636-5641; Stein et al. (1998) Genes Dev. 12:667-678).

The physiological role of ephrins and Eph receptors lies primarily in the formation and organization of the vasculature (McBride et al. (1998) Mech. Dev. 77, 201-204; Wang et al. (1998) Cell 93:741-753) and the patterning of topographic maps in the developing retinotectal and central nervous systems, where Eph signaling transmits cues for axon guidance (Drescher et al. (1995) Cell 82:359-370; Nakamoto et al. Cell 86:755-766; Knoll et al. (2002) Trends Neurosci. 25:145-149; Rashid et al. (2005) Neuron 47:57-69). Furthermore, some Eph receptors, including EphA2 and EphB2 (Nakada et al. (2004) Cancer Res. 64:3179-3185), have been implicated in tumorigenesis. Specifically, the EphA2 receptor is overexpressed and functionally important in cancers of the breast (Zelinski et al. (2001) Cancer Res. 61:2301-2306; Wu et al. (2004) Pathol. Oncol. Res. 10:26-33), prostate (Walker-Daniels et al. (1999) Prostate 41:275-280; Zeng et al. (2003) Am. J. Pathol. 163:2271-2276), brain (Wykosky et al. (2005) Mol. Cancer Res. 3:541-551; Hatano et al. (2005) Neoplasia. 7:717-722), and ovary (Thaker et al. (2004) Clin. Cancer Res. 10:5145-5150).

Due to the absence of the receptor in normal brain, opportunities exist for EphA2-targeted therapies based on ephrinA1, similar in high GBM cell molecular-specificity to what we have shown previously for another glioma-associated antigen, IL-13 receptor alpha-2 (Debinski et al. (1998) Nat. Biotechnol. 16:449-453). Interestingly, the ligand for EphA2, ephrinA1, is on average expressed (i) at lower levels when the receptor is elevated, and (ii) at higher levels when the receptor is low. Hence, we hypothesized that ephrinA1 is a tumor-suppressing protein in several solid tumors. Furthermore, a soluble recombinant homodimer, ephrinA1-Fc, activates EphA2 in GBM and other tumor cells, resulting in alteration of their malignant features. However, the prevailing notion has been that ephrinA1 must be anchored to the plasma membrane and form a homodimer in order to activate EphA2 in malignancy.

Previously, the tumor-suppressing potential of ephrinA1 has been demonstrated using, e.g., a recombinant, covalently-linked homodimeric ephrinA1-Fc. This protein activates and reverses the oncogenic properties of EphA2 in tumor cells in culture, causing receptor down-regulation, cell morphology changes, suppression of integrin function, and negative regulation of invasion, migration, and anchorage-independent growth (Wykosky et al. (2005) Mol. Cancer Res. 3:541-551); (Miao et al. (2000) Nat. Cell Biol. 2:62-69; Carter (2002) Nat. Cell Biol. 4:565-573; Walker-Daniels et al. (2002) Mol. Cancer Res. 1:79-87; Duxbury et al. (2004) Biochem. Biophys. Res. Commun. 320:1096-1102). The known physiological roles of ephrinA1, also thought to be mediated by a membrane-anchored form of the ligand and dependent on cell-cell contact, include induction of cell repulsion and growth cone collapse during central nervous system development (Marquardt et al. (2005) Cell 121:127-139). In addition, ephrinA1 has been shown to be expressed in the developing vasculature during embryogenesis (McBride et al. (1998) Mech. Dev. 77:201-204), induces endothelial cell migration (Pandey et al. (1995) Science 268:567-569) and the formation of capillary-like structures *in vitro* (Daniel et al. (1996) Kidney Int. Suppl 57:S73-S81), and plays a role in angiogenesis and neovascularization *in vivo* (Cheng et al. (2002) Mol. Cancer Res. 1:2-11).

EphrinA1 was originally isolated as a TNF-α-inducible, immediate-early response gene product from normal human umbilical vein endothelial cells (Holzman et al. (1990) Mol. Cell Biol. 10:5830-5838). The full-length mature protein is composed of 187 amino acids with a molecular weight of 22 kDa. The C-terminus of ephrinA1, due to its hydrophobic nature interrupted by several charged amino acids extending to the extreme C-terminal end, has high structural similarity to GPI-linked, membrane-anchored proteins (Ferguson et al. (1988) Annu. Rev. Biochem. 57:285-320). Interestingly, however, the original study did not provide evidence for its presence on the surface of normal endothelial cells. It was only later demonstrated that the ligand may exist as a GPI-linked protein in ephrinA1-transfected human embryonic kidney and breast cancer cells by its release into the media upon proteolytic cleavage with phosphatidylinositol-specific phospholipase C (PI-PLC) (Shao et al. (1995) J. Biol. Chem. 270:5636-5641). Additional indirect support for the membrane-anchored presence of the ligand was in the finding that soluble ephrinA1 could activate the EphA5 receptor only when clustered by antibodies against C-terminal epitope tags (Davis et al. (1994) Science 266:816-819).

These observations, coupled with structural studies on active Eph/ephrin complexes (Himanen et al. (2001) Nature 414, 933-938; Toth et al. (2001) Dev. Cell 1:83-92), gave rise to the notion that clustering of ephrins is a process necessary for Eph receptor activation that can be accomplished in a number of ways: via membrane attachment (Davis et al. (1994) Science 266:816-819; Shao et al. (1995) J. Biol. Chem. 270:5636-5641), antibody-mediated clustering (Davis et al. (1994) Science 266:816-819), or the formation of soluble homodimers through disulfide bonding of an IgG-Fc conjugate (Stein et al. (1998) Genes Dev. 12:667-678). Hence, membrane-bound ephrinA1 is considered the endogenous, functional form of the ligand (Beckmann et al. (1994) EMBO J. 13:3757-3762; Xu et al. (1997) J. Mol. Med. 75:576-586; Kullander et al. (2002) Nat. Rev. Mol. Cell Biol. 3:475-486; Pasquale (2005) Nat. Rev. Mol. Cell Biol. 6:462-475). The majority of studies on the role of ephrinA1 and other ephrinA's, both in physiology and in tumorigenesis, employ the Fc-conjugated dimeric forms of the ligand, often pre-clustered by the addition of IgG (Davis et al. (1994) Science 266:816-819; Daniel et al. (1996) Kidney Int. Suppl 57:S73-S81).

There remains a need to simplify and more efficiently utilize ephrinA1, e.g., for its anti-tumor activity.

### Summary of the Invention

The present invention is ephrinA1 in monomeric form for the use of claim. The ephrinA1 is preferably isolated, recombinant ephrinA1.

A composition may comprise, consist of or consist essentially of ephrinA1 in monomeric form in (and preferably solubilized in) a pharmaceutically acceptable carrier, e.g., an aqueous carrier.

A method of detecting a cancer tumor expressing the oncogenic receptor EphA2 in a subject, may comprise: administering ephrinA1 in monomeric form to said subject, wherein said ephrinA1 is coupled to a detectable group, and then detecting said detectable group at said tumor in said subject.

The present invention provides EphrinA1 in monomeric form for use in treating a cancer expressing the oncogenic receptor EphA2 in a subject, wherein said ephrinA1 is coupled to a therapeutic agent.

A method of treating cancer in a subject in need thereof, may comprise administering a first compound that specifically binds to Eph receptors to said subject in a treatment effective amount, wherein said first compound is coupled to a first therapeutic agent, and concurrently administering a second compound that specifically binds to IL13 receptors (IL13R) to said subject in a treatment effective amount, wherein said second compound is coupled to a second therapeutic agent. In some embodiments, the Eph receptors are EphA2, and the IL13 receptors are IL13Rα2.

A further aspect of the present invention is the use of ephrinA1 as described herein, or the combination of such ephrinA1 and another therapeutic agent such as IL-13 (and its mutants) as described herein, for the preparation of a medicament for use in treating a cancer as described herein.

The foregoing and other objects and aspects of the present invention are explained in greater detail in the specification set forth below.

### Brief Description of the Drawings

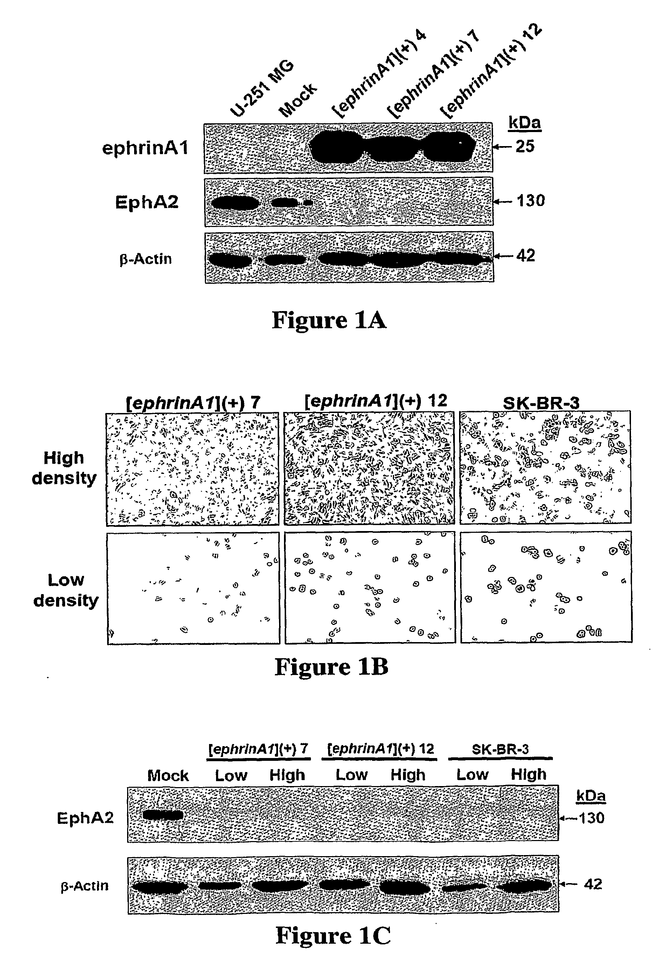

**Figures 1A-1C****.** EphrinA1 and EphA2 expression in GBM and breast cancer cells in the presence or absence of cell-cell contact. **Figure 1A****.** Western blot of confluent parental, mock- and *ephrinA1*-transfected U-251 MG GBM cells. Three individual ephrinA1-expressing clones are shown (#4., #7, #12). **Figure 1B**. Light microscopy of U-251[*ephrinA1*](+) #7, #12, and SK-BR-3 breast cancer cells plated at high and low density. **Figure 1C****.** Western blot of EphA2 in high density mock-transfected cells and in low and high density U-251[*ephrinA1*](+) #7, #12, and SK-BR-3 cells.

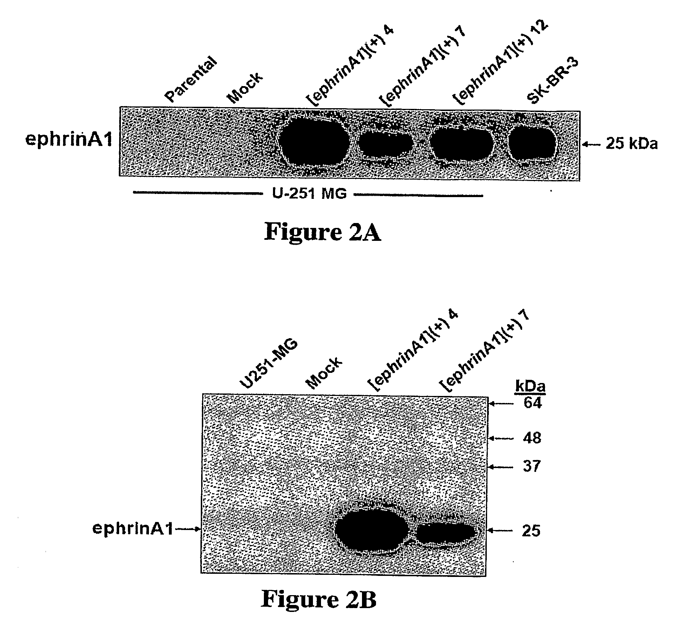

**Figures 2A-2B****.** EphrinA1 expression in conditioned media under reducing and non-reducing conditions. **Figure 2A****.** Western blot of ephrinA1 immunoreactivity in media obtained from parental U-251 MG, mock, U-251 [*ephrinA1*](+) #4, #7, #12, and SK-BR-3 cells, all subject to reducing conditions. **Figure 2****B.** Western blot of ephrinA1 immunoreactivity in media obtained from parental U-251 MG, mock, and U-251[*ephrinA1*](+) #4 and #7 cells under non-reducing conditions.

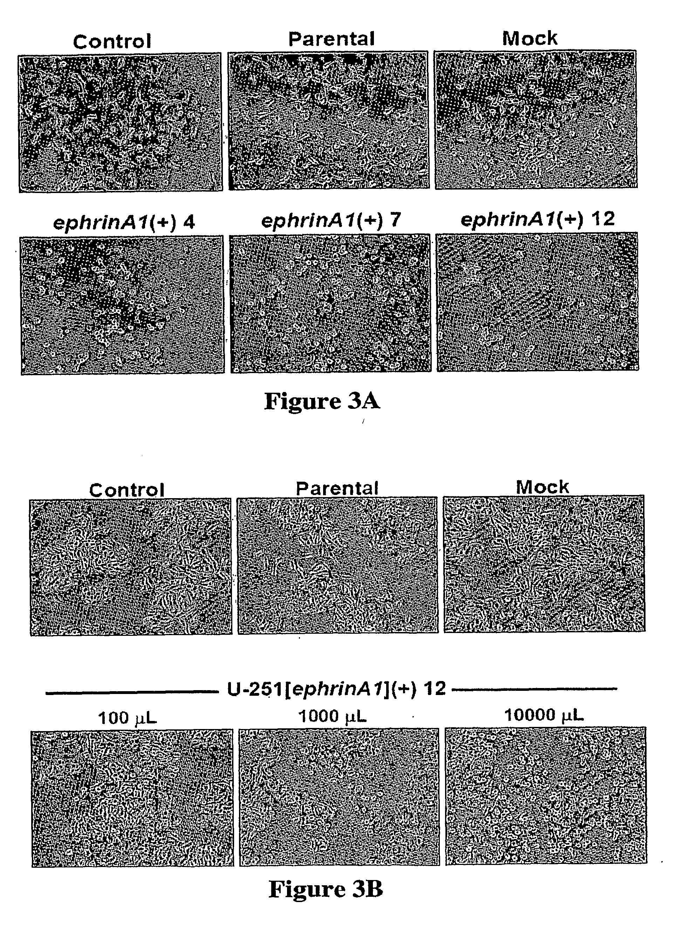

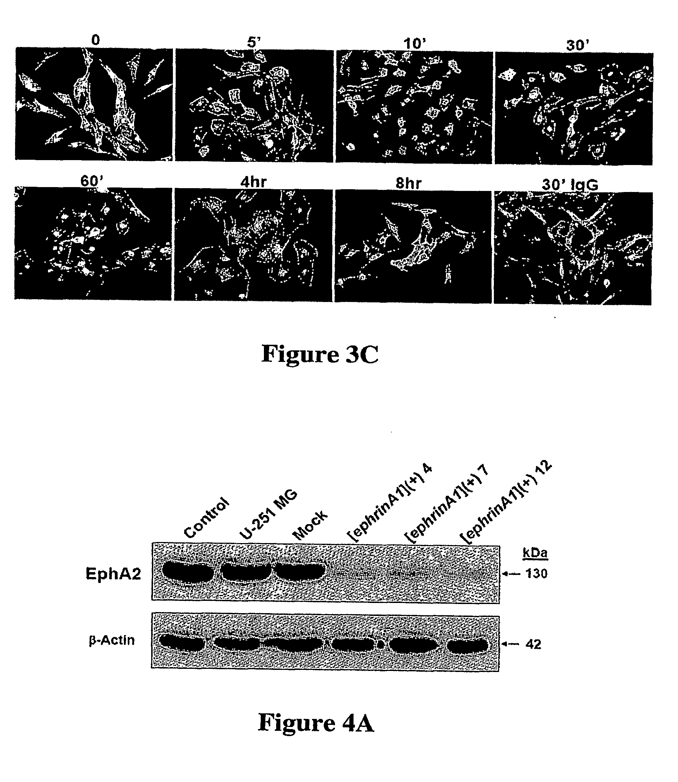

**Figures 3A-3C****.** Effect of ephrinA1-conditioned media and ephrinA1-Fc on GBM cell morphology. **Figure 3A****.** Light microscopy of U-251 MG cells after treatment for 30 min with conditioned media obtained from parental, mock, and U-251[*ephrinA1*](+) #4, #7, and #12 cells. **Figure 3****B.** Light microscopy of U-251 MG cells after treatment for 15 min with U-251[*ephrinA1*](+) #12 media. 100 µL or 1000 µL of conditioned media was diluted to a final volume of 10mL with serum-free growth media or undiluted (10000 µL). Parental and mock-conditioned media were undiluted. **Figure 3C****.** F-actin staining of U-251 MG cells treated with 1 µg/mL of ephrinA1-Fc or IgG control for the indicated times.

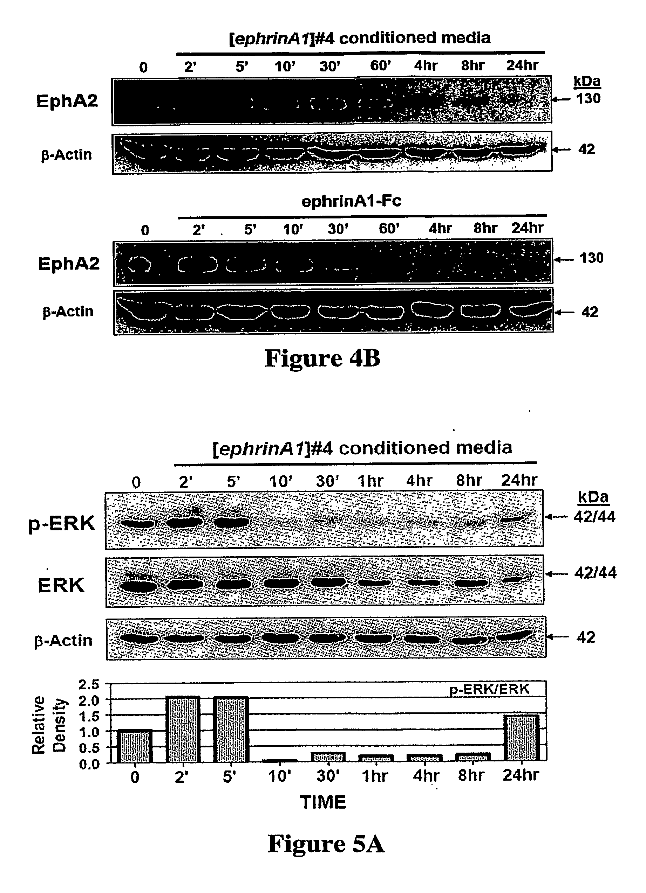

**Figures 4A-4B****.** EphA2 expression in response to treatment with ephrinA1-conditioned media or ephrinA1-Fc. **Figure 4A****.** Western blot of EphA2 immunoreactivity in U-251 MG cells treated for 1hr with conditioned media from parental U-251 MG, mock, and U-251[*ephrinA1*](+) #4, #7, and #12 cells. **Figure 4****B.** Western blot of EphA2 immunoreactivity in U-251 MG cells treated with U-251 [*ephrinA1*](+) #4 media or 1 µg/mL ephrinA1-Fc for the indicated times.

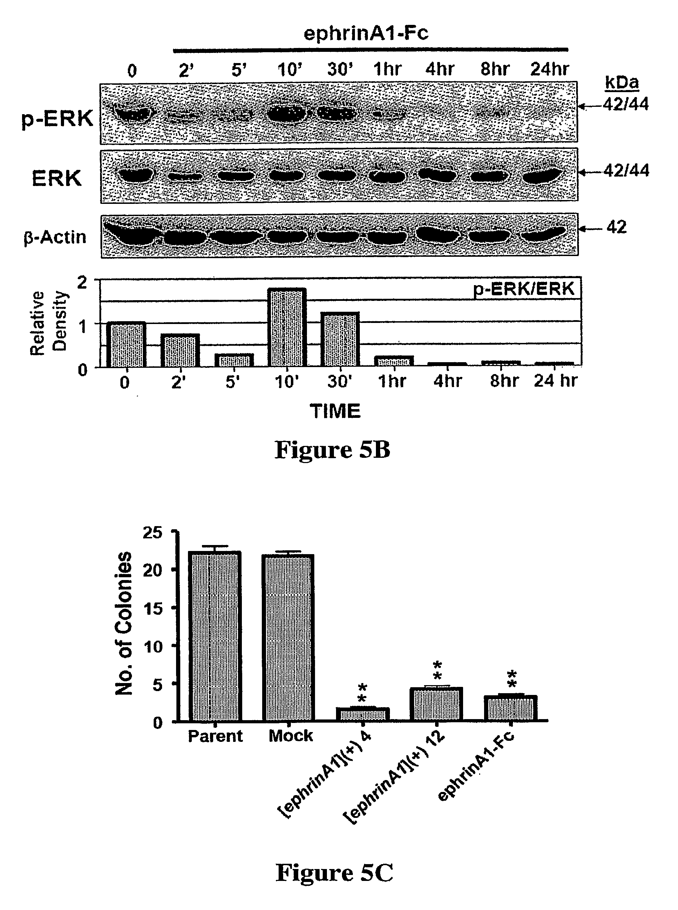

**Figures 5A-5C****.** Effect of soluble, monomeric ephrinA1 and ephrinA1-Fc on the RAS-MAPK pathway and anchorage-independent growth. **Figure 5A****.** Western blotting of total cell lysates for phosphorylated-ERK (p-ERK), total ERK, and β-Actin following treatment with media obtained from U-251 *ephrinA1*[+] #4 cells. **Figure 5****B.** Western blotting for p-ERK, total ERK, and β-action following treatment with 1 µg/mL ephrinA1-Fc. Densitometry revealed the fold change in p-ERK expression normalized to total ERK for each timepoint. **Figure 5C****.** Colony formation in soft agar of parental, mock-, and U-251*ephrinA1*[+] #4 and #12 cells as well as parental U-251 MG cells treated with 1 µg/mL ephrinA1-Fc. Colonies consisting of more than 50 cells were counted. **, p<0.001 *vs.* Mock, Bonferroni's Multiple Comparison Test.

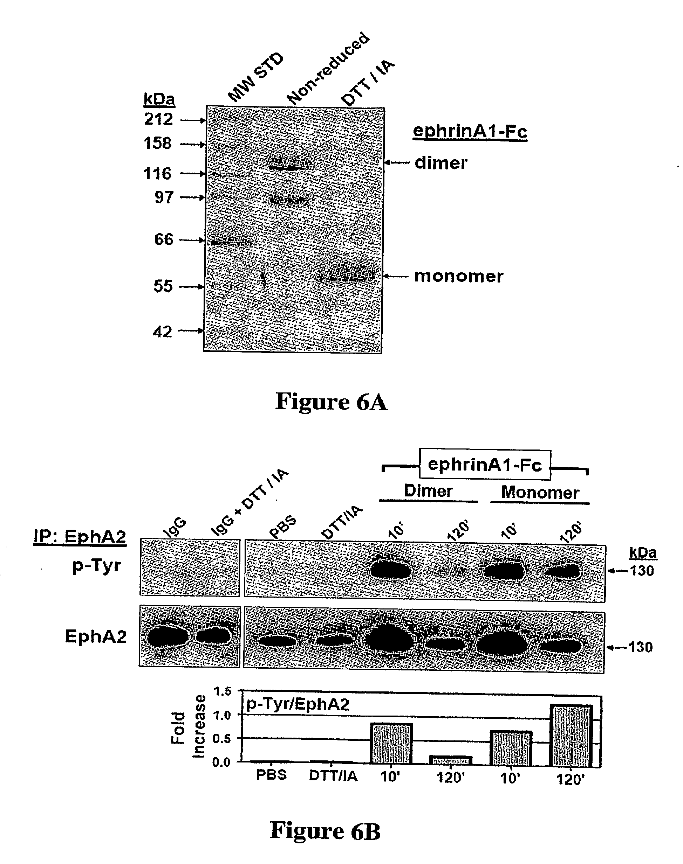

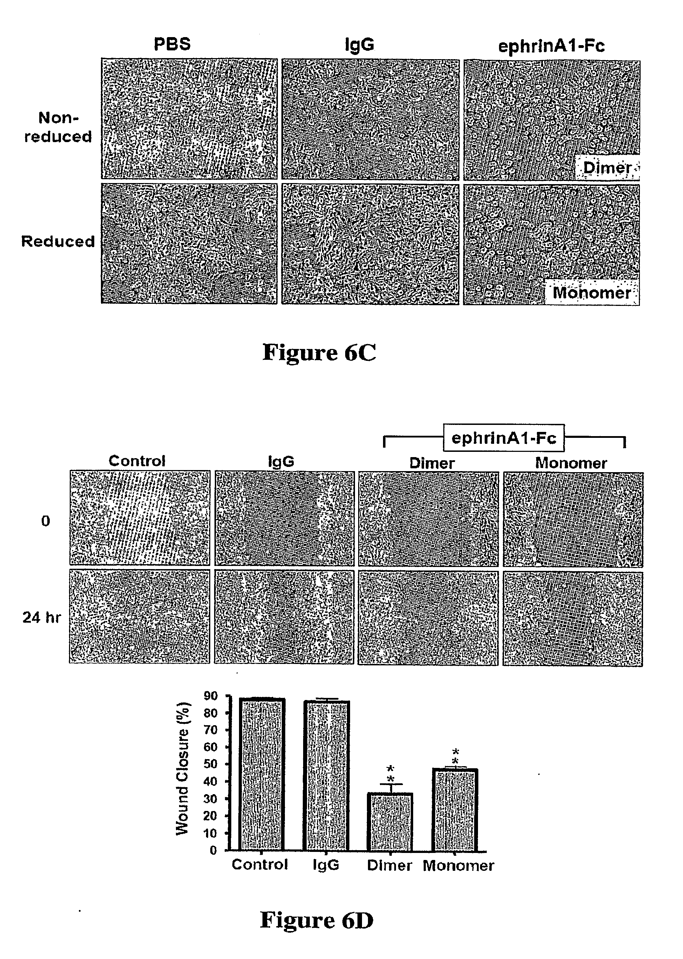

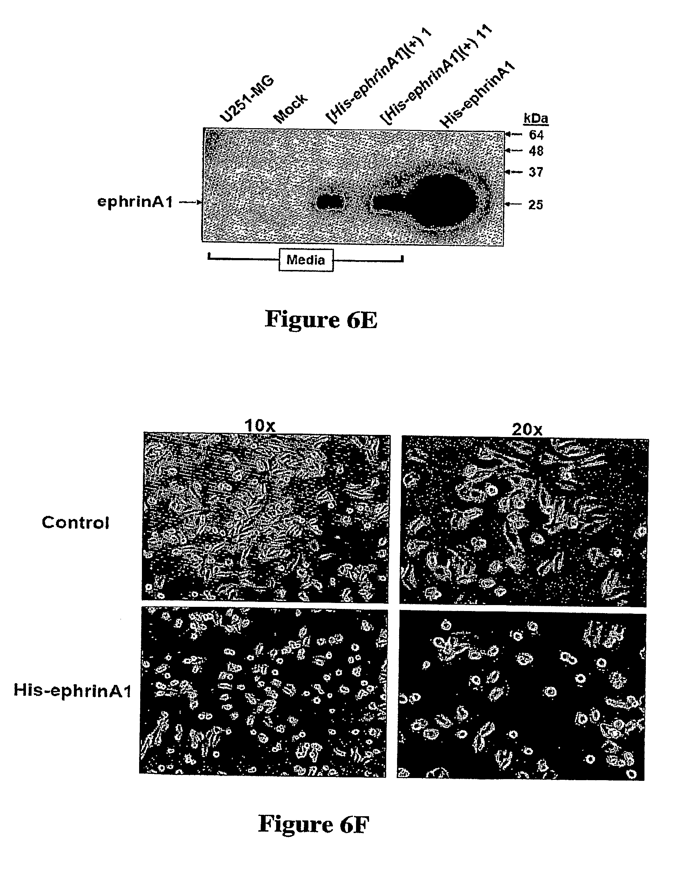

**Figures 6A-6F****.** Effect of monomeric ephrinA1 on U-251 MG cell morphology, EphA2 activation, and cell migration. **Figure 6A****.** SDS-PAGE analysis of non-reduced and reduced (DTT/IA) ephrinA1-Fc showing the relative sizes of dimeric versus monomeric ephrinA1-Fc used to treat U-251 MG cells. **Figure 6****B.** Phosphotyrosine (p-Tyr) and EphA2 detected by western blotting following immunoprecipitation with EphA2 in U-251 MG cells treated with dimeric or monomeric ephrinA1-Fc or IgG, PBS, or DTT/IA. Densitometry analysis revealed the relative density of phosphorylated EphA2 compared to total EphA2. **Figure 6C****.** Light microscopy of U-251 MG cells treated with 1 µg/mL dimeric or monomeric ephrinA1-Fc, IgG, or PBS. **Figure 6D****.** Migration assay measuring the percent wound closure over time of U-251 MG cells in the presence of dimeric or monomeric ephrinAl-Fc. **, p<0.001 *vs.* IgG-treated cells, Bonferroni's Multiple Comparison Test. **Figure 6E****.** Western blotting under non-reducing conditions of conditioned media from parental U-251 MG, mock-transfected, U-251[*His-ephrinA1*](+) #1 and #11 cells, and of pure His-ephrinA1 protein isolated from the conditioned media of [*ephrinA1*](+) #11 cells. **Figure 6F****.** Light microscopy of U-251 MG cells treated for 1 hour with less than 0.5 µg/mL His-ephrinA1 protein. Photographs taken with a 10x objective (left panels) or 20x objective lens (right panels). Controls were treated with an equal volume of vehicle (column elution buffer).

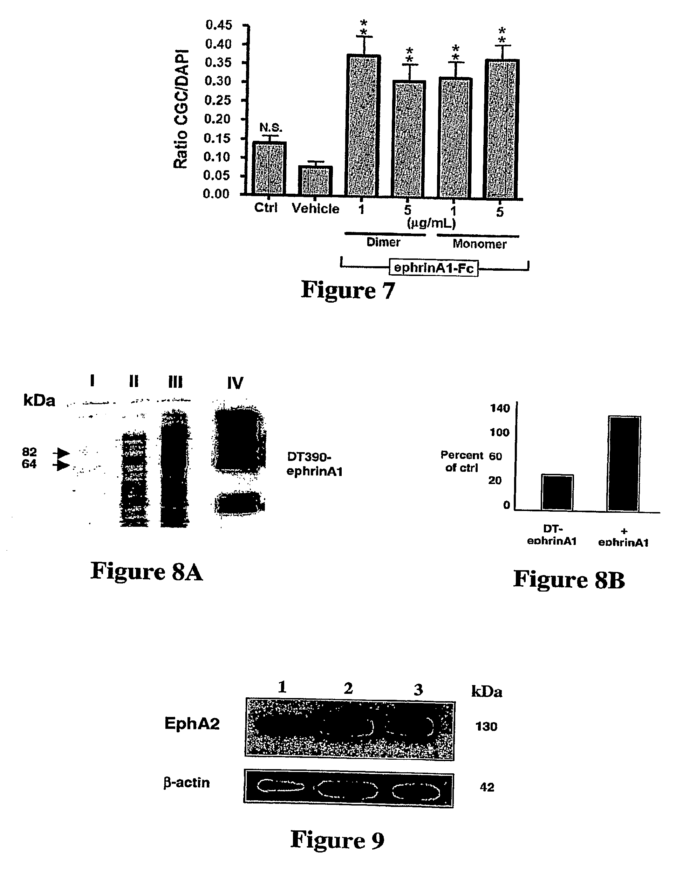

**Figure 7****.** Effect of monomeric and dimeric ephrinA1 on neuronal growth cones. Graphical representation of collapsed neuronal growth cones/total cell number (CGC/DAPI) for each treatment group. Primary rat cortical neurons were stained for F-actin following treatment for 1 hour with 1 µg/mL of a monomer or dimer of ephrinA1-Fc or an equal volume of PBS (vehicle). Nuclei were stained with DAPI. Control, non-treated cells. **, p<0.001 *vs.* vehicle; N.S., not statistically significant versus vehicle, p>0.05, Newman-Keuls Multiple Comparison Test.
**Figure 8A-8B****.** DT390-ephrinA1 recombinant protein expression in *E. coli* and partial purification. **Figure 8A****.** DT390-ephrin A1 is a major bacterial protein upon induction with IPTG. *I,* molecular size markers; *II,* pre-induced lysated of cells; *III,* IPTG-induced production of DT390-ephrin A1 in bacteria (lanes I to III represent SDS-PAGE); *IV,* western blot using anti-ephrinAl antibody of partially purified DT39D-ephrinA1 cytotoxin. **Figure 8****B.** Cell proliferation assay in U-215 MG GBM cells using DT390-ephrinA1 (15 nM) in the absence or presence of an excess of ephrinA1-Fc.
**Figure 9****.** Transient silencing of EphA2 with siRNA. Western blot of EphA2 in U-251 MG cells treated with *ephA2* siRNA (*1*), nonsense siRNA (*2*), and sham-treated (*3*).

### Detailed Description

The present invention is explained in greater detail below. This description is not intended to be a detailed catalog of all the different ways in which the invention may be implemented, or all of the features that may be added to the instant invention. For example, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure which do not depart from the instant invention. Hence, the following specification is intended to illustrate some particular embodiments of the invention, and not to exhaustively specify all permutations, combinations and variations thereof.

As used in the description of the invention and the appended claims, the singular forms "a", "an" and "the" are intended to include the plural forms as well, unless the context clearly indicates otherwise. Furthermore, the term "about," as used herein when referring to a measurable value such as an amount of a compound, dose, time, temperature, and the like, is meant to encompass variations of 20%, 10%, 5%, 1%, 0.5%, or even 0.1% of the specified amount. Also, as used herein, "and/or" refers to and encompasses any and all possible combinations of one or more of the associated listed items, as well as the lack of combinations when interpreted in the alternative ("or").

We have previously demonstrated that the EphA2 receptor tyrosine kinase is an attractive molecular target in glioblastoma multiforme (GBM), the most common and lethal primary malignant brain tumor. The expression of this receptor has been directly linked to poor cancer patient survival in GBM, as well as in breast and prostate carcinomas. See, e.g., U.S. Patent Application Publication 2006/0121539 (Debinski et al.).

We have now uncovered that ephrinA1, a ligand for the EphA2 receptor, can be released into the extracellular environment as a monomeric protein by both *ephrinA1*-transfected cancer cells and cancer cells that endogenously express the ligand. This monomer of ephrinA1 retains its functional activity towards EphA2 in conditioned media and after purification from the media of ephrinA1-expressing cells. In addition, a recombinant Fc-fusion ephrinA1 reduced to its monomeric form exhibited tumor-suppressing properties by activating the EphA2 oncoprotein, which leads to anti-oncogenic signaling and down-regulation of the receptor. Released as a monomer, ephrinA1 elicits profound changes in cancer cell morphology, suppresses the oncogenic Ras-MAPK signaling pathway, and impairs migration and anchorage-independent growth. These data indicate that monomeric ephrinA1 is a tumor-suppressing protein in GBM and in other cancers, such as of the breast. Further, a monomer of ephrinA1 effectively augments neuronal growth cone collapse, suggesting that this monomeric form of the ligand is utilized in modulating developmental processes as well.

EphrinA1 can decrease the tumorigenic potential of GBM and other cancer cells through the activation and subsequent down-regulation of the EphA2 oncoprotein, which has been previously observed using a recombinant, dimeric form of ephrinA1-Fc and/or cells expressing ephrinA1 in co-culture with those expressing EphA2 (Wykosky et al. (2005) Mol. Cancer Res. 3:541-551; Walker-Daniels et al. (2002) Mol. Cancer Res. 1, 79-87; Carles-Kinch et al. (2002) Cancer Res. 62:2840-2847; Duxbury et al. (2004) Oncogene 23:1448-1456). The function of ephrinA1 has been studied in this manner because this protein, like other members of the ephrin family, is considered a membrane-anchored ligand requiring cell-cell contact or oligomerization/clustering, if soluble, in order to mimic its membrane-bound nature and fulfill the structural requirements for activation of the cognate Eph receptor.

The Eph receptors comprise the largest family of tyrosine kinase receptors, a group of transmembrane proteins that are crucial in mediating important signal transduction pathways in cells such as those controlling growth, migration, and differentiation. The fourteen members of the Eph receptors are divided into A and B classes based on the similarity of their extracellular domains and their ability to interact with their membrane-bound ligands, the ephrins.

The notion that Ephs and ephrins may not, in fact, require the formation of oligomeric complexes in physiology has recent precedence in the study of this family of proteins in the context of normal nervous system development. Another member of the ephrinA family, ephrinA5, can engage in a functional interaction with the EphB2 receptor in a 1:1 heterodimeric complex, both in crystal structures and in solution, even at exceedingly high concentrations (Himanen et al. (2004) Nat. Neurosci. 7:501-509). This 1:1 ephrinA/EphB interaction is in contrast to the heterotetrameric and higher-order oligomeric complexes thought to be necessary for full Eph receptor activation and function (Davis et al. (1994) Science 266:816-819; Himanen et al. (2001) Nature 414:933-938; Toth et al. (2001) Dev. Cell 1:83-92; Bartley et al. (1994) Nature 368:558-560). In addition, it represents one of the few known instances of cross-talk between A and B class. Ephs and ephrins, which was in fact cited as a possible explanation for the existence of the unexpected, functional 1:1 receptor/ligand complex (Himanen et al. (2004) Nat. Neurosci. 7:501-509).

We demonstrate for the first time that monomeric ephrins play a role as soluble factors in activating Ephs of their own class during cancer maintenance and/or progression and normal developmental/physiological processes. This has simplifying implications on the design of therapies against solid tumors exploiting the ephrinA1/EphA2 system.

In support of this finding, 12-amino acid ephrinA1-mimetic peptides have the capacity to bind and activate EphA2 (Koolpe et al. (2002) J. Biol. Chem. 277:46974-46979; U.S. Patent Publication No. 2004/0180823). The peptides compete with other ephrinA ligands for binding to the receptor, which may indicate that they utilize the ephrinA binding sites of EphA2. The amino acid sequences of these peptides do not show homology to ephrinA1.

The experiments with a homodimer of ephrinA1-Fc reduced to a monomeric form, as well as the monomer of His-ephrinA1 isolated from conditioned media, directly reveal the functional activity of this ligand as a non covalently-linked single-chain protein. This, together with the finding that soluble, monomeric ephrinA1 is present in the media of GBM and breast cancer cells and exhibits functional properties similar to dimeric ephrinA1-Fc, suggests that the ligand is capable of interacting with EphA2 in a manner that is not dependent on juxtacrine interactions.

Our results demonstrating that ephrinA1 as a non covelently-linked monomer is functional in eliciting collapse of neuronal growth cones support a role for the monomer in physiology. The role of dimeric or clustered ephrinA ligands in affecting this process has been established previously (Marquardt et al. (2005) Cell 121:127-139; Meima et al. (1997) Eur. J. Neurosci. 9:177-188). With respect to development, it is well-known that a major function of Eph/ephrin interaction is the formation of boundaries between populations of cells, such as with respect to the developing retinotectal system (Nakamoto et al. (1996) Cell 86:755-766; Rashid et al. (2005) Neuron 47:57-69; Xu et al. (1997) J. Mol. Med. 75:576-586). While processes such as these may be in part dependent on cell-cell contact, our results point to a paracrine role for ephrinA1, both in physiology and in cancer.

The role of ephrinA1 as a tumor-suppressing protein in GBM was first suspected based on our previous findings that the ligand was largely absent in the majority of GBM cases, in sharp contrast to abundantly overexpressed EphA2 (Wykosky et al. (2005) Mol. Cancer Res. 3:541-551). This expression pattern, coupled with the ability of recombinant homodimeric ephrinA1-Fc to decrease invasion and anchorage-independent growth, pointed to the ligand as a potentially not-sufficiently-increased-during-oncogenesis tumor-suppressing protein in GBM. A similar pattern of differential EphA2/ephrinA1 expression has been shown in breast cancer (Macrae et al. (2005) Cancer Cell 8:111-118), and ephrinA1 can possess these same tumor-suppressing properties in several other solid tumors (Walker-Daniels et al. (2002) Mol. Cancer Res. 1:79-87; Carles-Kinch et al. (2002) Cancer Res. 62:2840-2847; Duxbury et al. (2004) Oncogene 23:1448-1456). Presumably, under normal circumstances, ephrinA1 activates EphA2 in pre-malignant and malignant cells, maintaining the expression of the receptor at a low level and, by this, suppressing oncogenic signaling pathways. In support of this hypothesis, ephrinA1-mediated activation of EphA2 has been shown to suppress the activity of ERK in breast and prostate cancer (Miao et al. (2001) Nat. Cell Biol. 3:527-530) as well as a two-stage model of skin carcinogenesis (Guo et al. (2006) Cancer Res. 66:7050-7058). Here, we have found similar phenomena in GBM cells.

Therefore, EphA2 could play a dual role in cancer: anti-oncogenic when activated by tumor-suppressing ephrinA1 and maintained at low levels, and oncogenic when ephrinA1 is not present and thus contributing to its own overexpression that is driven by other mechanisms. In fact, the oncogenicity of un-activated, non-tyrosine-phosphorylated EphA2 has been shown directly in its ability to transform mammary epithelial cells (Zelinski et al. (2001) Cancer Res. 61:2301-2306), which may be attributed to its ligand-independent kinase activity (Zantek et al. (1999) Cell Growth Differ. 10:629-638). Additional support for ephrins as potential tumor-suppressing proteins acting through Eph receptors is contained in a recent study that shows the . EphB4 receptor as a tumor suppressor in breast cancer via stimulation with ephrinB2, a ligand which is absent in the majority of breast cancer cells (Noren et al. (2006) Nat. Cell Biol. 8:815-825). Interestingly, ephrinAl has also been shown to play a role in angiogenesis and neovascularization through its effect on endothelial cells expressing EphA receptors (Daniel et al. (1996) Kidney Int. Suppl 57:S73-S81; Cheng et al. (2002) Mol. Cancer Res. 1:2-11). However, these observations point toward a possible pleiotropic effect of ephrinA1 with respect to cell-type and specific environment.

The form of ephrinA1 required or able to exert tumor-suppressing functions through EphA2 has not been previously established. Covalently-linked dimers or clustered forms of the ligand have been shown to activate the receptor, which results in decreased tumorigenic potential, *both in vitro* and *in vivo* (Noblitt et al. (2004) Cancer Gene Ther. 11:757-766). We show that soluble, monomeric ephrinA1 exhibits a full spectrum of biological activities. Furthermore, we show that ephrinA1 is not dependent on juxtacrine interactions and can, indeed, function in a paracrine manner. These findings will aid in deciphering the role of ephrinA1 and EphA2 in solid tumor progression. In addition, they will facilitate the design and allow for a wider application of ephrinAl-based therapeutics targeting the EphA2 receptor involving, *inter alia,* the use of simplified soluble recombinant proteins and viral gene therapy.

EphA2 is internalized when bound by its natural ligand, ephrin-A1 (Walker-Daniels et al. (2002) Mol Cancer Res 1:79-87) and thus represents an attractive target for therapies utilizing bacterial toxin-containing cytotoxins (Debinski W. (2002) Molecular "Targeting of Brain Tumors with Cytotoxin," In: Chimeric Toxins (Lorberboum-Galski & Lazarovici, eds., Harwood Academic Publishers) pp. 222-246; Debinski (2002) Cancer Invest. 20:801-809). Moreover, EphA2 can serve as a target for active immunotherapy in the form of anti-cancer vaccines (Hatano et al. (2005) Neoplasia 7:717-22; Alves et al. (2003) Cancer Res 63:8476-8480). Coincidentally, the same applies to IL13Rα2, which has become a target of interest in the design of anti-cancer vaccines (Okano et al. (2002) Clin Cancer Res 8:2851-2855). Thus, EphA2 is a novel target for prospective molecularly targeted therapies of GBM.

What makes the EphA2/ephrin A1 system different from the IL13 system in HGG is that the ephrinA1 ligand has a profound anti-tumor effect on cancer cells, and its peptidomimetics are being searched (Koolpe et al. (2002) J Biol Chem 277:46974-46979; U.S. Patent Publication No. 2004/0180823). Also, EphA2 is expressed in an additional compartment of HGG, the neo-vascular bed, which should provide a greater benefit when using anit-EphA2 targeted drugs. In addition, the recombinant drug candidates may be of use in many other solid tumors. Being that the heterogeneity of HGG is one the factors hampering the outcome of treatments, a possibility of having at least two specific molecular targets for therapeutic delivery of differing intratumoral distribution in this disease may be an advantage.

Recombinant cytotoxins consisting of a ligand targeted to a tumor-specific molecule and a bacterial toxin derivative are emerging as a way to improve the outcome of patients with GBM and other cancers. We have previously generated IL13-based, bacterial toxin-containing recombinant cytotoxic fusion proteins that have been shown to be very potent anti-glioma cytotoxins *in vitro* and *in vivo* (Debinski et al. (1999) Clin Cancer Res 5:985-990; Debinski et al. (1995) Clin Cancer Res 1:1253-1258; Debinski et al. (1998) Nature Biotech 16:449-453). The first generation of IL13-based cytotoxin, hIL13-PE38QQR, contains wild type IL13 and a derivative of the bacterial toxin Pseudomonas exotoxin A (PE). This cytotoxin has entered Phase III trials in patients with recurrent GBM.

Recombinant cytotoxins are: (a) water soluble and relatively small (50 to 200 kDa) compounds, (b) deliverable using an interstitial/intratumoral drug administration system termed convection enhanced delivery (CED) (Debinski (2002) Cancer Invest. 20:801-809; Laske et al. (1997) Nature Med 3:1362-1368), (c) very potent at killing cells: their IC₅₀s can be in the femtomolar range (Debinski et al. (1999) Clin Cancer Res 5:985-990), (d) virtually independent of anti-apoptotic cell predisposition (Keppler-Hafkemeyer et al. (1998) Biochemistry 37:16934-16942; Keppler-Hafkemeyer et al. (2000) Int J Cancer 87:86-94), (e) not readily resistant, and (f) cytotoxic in proportion to the number of internalized binding sites on cancer cells, and thus independent of any other role plasma membrane targets for cytotoxins may play in cancer pathogenesis (Pastan et al. (1992) Ann Rev Biochem 61:331-354). Recombinant cytotoxins represent a new qualitative group of anti-cancer therapeutics (Carglia et al. (2000) Eur J Biochem 267:3919-3936).

### 1. Definitions.

As used herein, "ephrin" or "ephrins" are those proteins, peptides, variants, and/or fragments thereof, belonging to the ephrin family of proteins, e.g., ephrinA1, ephrinA2, ephrinA3, ephrinA4, ephrinA5, ephrinB1, ephrinB2, and ephrinB3. In some embodiments, ephrins are ephrinA1 proteins or peptides. In some embodiments, ephrin molecules are mammalian (e.g., human or mouse) ephrinA1 proteins or peptides.

As used herein, the terms "Eph" or "Eph receptor" refer to a class of transmembrane receptor tyrosine kinases, thought to include at least eleven paralogous genes, though many more orthologs exist within this class, e.g. homologs from different species. Eph receptors, in general, are a discrete group of receptors related by homology. They are characterized by an extracellular domain containing a characteristic spacing of cysteine residues near the N-terminus, and two fibronectin type III repeats. Exemplary Eph receptors include EphA1, EphA2, EphA3, EphA4, EphA5, EphA6, EphA7, EphA8, EphB1, EphB2, EphB3, EphB4 and EphB5, eph, elk, eck, sek, mek4, hek, hek2, eek, erk, tyro1, tyro4, tyro5, tyro6, tyro11, cek4, cek5, cek6, cek7, cek8, cek9, cek10, bsk, rtk1, rtk2, rtk3, myk1, myk2, ehk1, ehk2, pagliaccio, htk, erk and nuk receptors. Furthermore, "mek4/sek receptors" refers to a closely related subgroup of the Eph receptor family, including the "mek4-related receptors" such as mek4, cek4, hek and tyro4; the "sek-related receptors" such as sek, cek8, pagliaccio, tyro1 and rtk1; and other phylogenetically related homologs such as eek, bsk, ehk1, ehk2, and cek7. In some embodiments, Eph receptors are the EphA2 receptors.

Ligands of the Eph receptors include, but are not limited to, the ephrins, such as those listed above. In some embodiments, ligands of the EphA2 receptors are ephrinA1 proteins or polypeptides. Further discussion of ephrins and Eph receptors is found in U.S. Patent Application Publication No. 2006/0121539 (Debinski et al.).

One of skill in the art will appreciate that analogues or fragments of ephrins will also specifically bind to the Eph receptors. Thus, the term "ephrin," when used in reference to a targeting molecule, also includes fragments, analogues or peptide mimetics of ephrins that also specifically bind to the Eph receptors (See, e.g., Pat. Publication Nos. 2006/0177452 and 2004/0180823 to Pasquale et al.).

"IL13" or "IL-13" as used herein refers to interleukin-13, which is a pleiotropic cytokine. IL13 has approximately 30% sequence identity with IL4 and exhibits IL4-like activities on monocytes/macrophages and human B cells (Minty et al. (1993) Nature, 362:248; McKenzie et al. (1987) Proc. Natl. Acad. Sci. USA, 90:3735). In particular, IL13 appears to be a potent regulator of inflammatory and immune responses. IL13 can up-regulate the monocyte/macrophage expression of CD23 and MHC class I and class II antigens, down-regulate the expression of Fc.gamma, and inhibit antibody-dependent cytotoxicity. IL13 can also inhibit nitric oxide production as well as the expression of pro-inflammatory cytokines (e.g. IL-1, IL-6, IL-8, IL-10 and IL-12) and chemokines (MIP-1, MCP), but enhance the production of IL-1.

Recombinant IL-13 is commercially available from a number of sources (e.g., R&D Systems, Minneapolis, Minn., USA, and Sanofi Bio-Industries, Inc., Tervose, Pa., USA). Alternatively, a gene or cDNA encoding IL-13 may be cloned into a plasmid or other expression vector and expressed in any of a number of expression systems according to methods well known to those of skill in the art. Methods of cloning and expressing IL-13 and the nucleic acid sequence for IL-13 are well known (see, for example, Minty et al. (1993) supra. and McKenzie (1987), supra). In addition, the expression of IL-13 as a component of a chimeric molecule is detailed below. Also contemplated is the use of specific IL-13 mutants as desribed in U.S. Patent No. 6,884,603 (Debinski et al.).

One of skill in the art will appreciate that analogues or fragments of IL-13 will also specifically bind to the IL-13 receptor. For example, conservative substitutions of residues (e.g., a serine for an alanine or an aspartic acid for a glutamic acid) comprising native IL-13 will provide IL-13 analogues that also specifically bind to the IL-13 receptor. Thus, the term "IL-13," when used in reference to a targeting molecule, also includes fragments, analogues or peptide mimetics of IL-13 that also specifically bind to the IL-13 receptor. Further discussion of IL13 as contemplated by the present invention can be found in U.S. Patent Nos. 5,328,984 (Pastan et al.), 5,614,191 (Puri et al.), 5,919,456 (Puri et al.), 6,296,843 (Debinski), 6,428,788 (Debinski et al.), 6,518,061 (Puri et al.), 6,576,232 (Debinski et al.), 6,630,576 (Debinski), and 6,884,603 (Debinski et al.).

"Recombinant" nucleic acid as used herein refers to a nucleic acid produced by combining two or more nucleic acid sequences from different sources, e.g., by use of molecular biology techniques, to form a new nucleic acid, e.g., a "heterologous" nucleic acid. The recombinant nucleic acid may be provided in the form of a "vector" or "delivery vector" in order to transform or transfect cells to contain the new nucleic acid. As used herein, a "vector" or "delivery vector" can be a viral or non-viral vector that is used to deliver a nucleic acid to a cell, tissue or subject.

A "recombinant" protein is a protein produced by a recombinant nucleic acid. The nucleic acid may or may not be inserted into the genome of a host cell. The nucleic acid may exist, e.g., in plasmid form in a host cell. Alternatively, the recombinant protein may be produced by in vitro translation of the recombinant nucleic acid.

As used herein, an "active" protein or peptide is one that retains at least one biological activity normally associated with that protein. Preferably, an "active" protein retains all of the activities possessed by the unmodified protein. By "retains" biological activity, it is meant that the polypeptide retains at least about 50%, 60%, 75%, 85%, 90%, 95%, 97%, 98%, 99%, or more, of the biological activity of the native protein (and can even have a higher level of activity than the native protein). A "non-active" protein or polypeptide is one that exhibits essentially no detectable biological activity normally associated with the polypeptide (e.g., at most, only an insignificant amount, e.g., less than about 10% or even 5%).

An "active fragment" of an ephrin protein of this invention is an amino acid sequence having fewer than all of the amino acids of the full or complete amino acid sequence of an ephrin, and that retains one or more of the activities associated with the ephrinA1 protein. For example, activities of the ephrinA1 protein include, but are not limited to, interacting with, binding to, and activating the EphA2 receptor.

A "biologically active fragment" or "active fragment" as used herein includes a polypeptide that comprises a sufficient number of amino acids to have one or more of the biological activities of the proteins or polypeptides of this invention. A fragment of a polypeptide of this invention can be produced by methods well known and routine in the art. Fragments of this invention can be produced, for example, by enzymatic or other cleavage of naturally-occurring proteins or polypeptides or by synthetic protocols that are well known. Such fragments can be tested for one or more of the biological activities of this invention according to the methods described herein, and/or according to any art-known and routine methods for identifying such activities. Such production and testing to identify biologically active fragments of the polypeptides described herein would be well within the scope of one of ordinary skill in the art and would be routine.

Fragments of the polypeptides of this invention may be at least about ten amino acids in length and retain one or more of the biological activities of the ephrin proteins. For example, non-homologous amino acid fragments that are 12 amino acids in length are active in EphA2 binding (Koolpe et al. (2002) J. Biol. Chem. 277:46974-46979).

An "isolated" protein or polypeptide means a protein or polypeptide that is separated or substantially free from at least some of the other components of the naturally occurring organism or virus, for example, the cell or viral structural components or other proteins or nucleic acids commonly found associated with the protein. As used herein, the "isolated" protein or polypeptide is at least about 25%, 30%, 40%, 50%, 60%, 70%, 75%, 80%, 85%, 90%, 95%, 97%, 98%, 99% or more pure (w/w).

As used herein an "isolated" cell is a cell that is free or substantially free from at least some of the other components of the naturally occurring organism. An "isolated" cell can be a cultured cell. Alternatively, an "isolated" cell can be a cell in a pharmaceutical composition. Further, an "isolated" cell can be a cell that has been implanted into a recipient host. According to this embodiment, the cell can be derived from the host subject or can be foreign to the subject.

By the term "express," "expresses" or "expression" of a nucleic acid coding sequence, in particular an ephrinA1 coding sequence, it is meant that the sequence is translated into a protein or polypeptide of ephrinA1. Prokaryotic and eukaryotic expression systems may each be employed according to standard techniques. The most common prokaryotic organism used for protein expression is *E. coli.* Eukaryotic expression may be desirable when post-translational processing and protein folding that are specific to eukaryotic cells is desired, e.g., glycosylation. Three common eukaryotic expression systems are yeast cells, insect cells, and mammalian cells.

To modify the ephrinA1 amino acid sequences disclosed herein or otherwise known in the art, amino acid substitutions can be based on any characteristic known in the art, including the relative similarity or differences of the amino acid side-chain substituents, for example, their hydrophobicity, hydrophilicity, charge, size, and the like. In particular embodiments, conservative substitutions (i.e., substitution with an amino acid residue having similar properties) are made in the amino acid sequence encoding ephrinA1.

In making amino acid substitutions, the hydropathic index of amino acids can be considered. The importance of the hydropathic amino acid index in conferring interactive biologic function on a protein is generally understood in the art (Kyte and Doolittle (1982) J. Mol. Biol. 157:105). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

Each amino acid has been assigned a hydropathic index on the basis of its hydrophobicity and charge characteristics (Kyte and Doolittle (1982) J. Mol. Biol. 157:105), and these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is also understood in the art that the substitution of amino acids can be made on the basis of hydrophilicity. U.S. Patent No. 4,554,101 teaches that the greatest local average hydrophilicity of a protein, as governed by the hydrophilicity of its adjacent amino acids, correlates with a biological property of the protein.

As detailed in U.S. Patent No. 4,554,101, the following hydrophilicity values have been assigned to amino acid residues: arginine (+3.0); lysine (±3.0); aspartate (+3.0 ± 1); glutamate (+3.0 ± 1); serine (+0.3); asparagine (+0.2); glutamine (+0.2); glycine (0); threonine (-0.4); proline (-0.5 ± I); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

Expression vectors can be designed for expression of proteins or polypeptides in prokaryotic or eukaryotic cells. For example, polypeptides can be expressed in bacterial cells such as *E. coli,* insect cells (e.g., in the baculovirus expression system), yeast cells or mammalian cells. Suitable host cells are discussed further in Goeddel, Gene Expression Technology: Methods in Enzymology 185, Academic Press, San Diego, Calif. (1990). Examples of vectors for expression in yeast *S. cerevisiae* include pYepSecl (Baldari et al., (1987) EMBO J. 6:229-234), pMFa (Kurjan and Herskowitz, (1982) Cell 30:933-943), pJRY88 (Schultz et al., (1987) Gene 54:113-123), and pYES2 (Invitrogen Corporation, San Diego, Calif.). Baculovirus vectors available for expression of nucleic acids to produce proteins in cultured insect cells (*e.g*., Sf 9 cells) include the pAc series (Smith et al., (1983) Mol. Cell. Biol. 3:2156-2165) and the pVL series (Lucklow, V.A., and Summers, M.d. (1989) Virology 170:31-39).

Vectors can be introduced into prokaryotic or eukaryotic cells via conventional transformation or transfection techniques. As used herein, the terms "transformation" and "transfection" refer to a variety of art-recognized techniques for introducing foreign nucleic acids (e.g., DNA) into a host cell, including calcium phosphate or calcium chloride co-precipitation, DEAE-dextran-mediated transfection, lipofection, electroporation, microinjection, DNA-loaded liposomes, lipofectamine-DNA complexes, cell sonication, gene bombardment using high velocity microprojectiles, and viral-mediated transfection. Suitable methods for transforming or transfecting host cells can be found in Sambrook et al. (Molecular Cloning: A Laboratory Manual, 2nd Edition, Cold Spring Harbor Laboratory press (1989)), and other laboratory manuals.

"Subjects" as used herein are generally human subjects and includes, but is not limited to, cancer patients. The subjects may be male or female and may be of any race or ethnicity, including, but not limited to, Caucasian, African-American, African, Asian, Hispanic, Indian, etc. The subjects may be of any age, including newborn, neonate, infant, child, adolescent, adult, and geriatric. Subjects may also include animal subjects, particularly mammalian subjects such as canines, felines, bovines, caprines, equines, ovines, porcines, rodents (e.g. rats and mice), lagomorphs, primates (including non-human primates), etc., screened for veterinary medicine or pharmaceutical drug development purposes.

Cancers that can be detected and/or treated by the compounds, compositions and methods described herein include those malignancies deprived of ephrinA1 and having increased levels of EphA2, its receptor, which is an oncogenic receptor. Examples of such cancers include, but are not limited to, breast cancer, bladder cancer, pancreatic cancer, brain cancer such as gliomas (e.g., GBM), etc.

"Therapeutic agent" as used herein may be any therapeutic agent including, but not limited to, radionuclides, chemotherapeutic agents, and cytotoxic agents. *See, e.g.,* U.S. Patent No. 6,949,245 to Sliwkowski.

"Radionuclide" as described herein includes, but is not limited to, ²²⁷Ac, ²¹¹At, ¹³¹Ba ⁷⁷Br, ¹⁰⁹Cd, ⁵¹Cr, ⁶⁷Cu, ¹⁶⁵Dy, ¹⁵⁵Eu, ¹⁵³Gd, ¹⁹⁸Au, ¹⁶⁶Ho, ^{113m}In, ^{115m}In, ¹²³I, ¹²⁵I, ¹³¹I, ¹⁸⁹Ir, ¹⁹¹Ir, ¹⁹²Ir, ¹⁹⁴Ir. ⁵²Fe, ⁵⁵Fe, ⁵⁹Fe, ¹⁷⁷Lu, ¹⁰⁹Pd, ³²P, ²²⁶Ra, ¹⁸⁶Re, ¹⁸⁸Re, ¹⁵³Sm, ⁴⁶Sc, ⁴⁷Sc, ⁷²Se, ⁷⁵Se, ¹⁰⁵Ag, ⁸⁹Sr, ³⁵S, ¹⁷⁷Ta, ¹¹⁷mSn, ¹²¹Sn, ¹⁶⁶Yb, ¹⁶⁹Yb, ⁹⁰Y, ²¹²Bi, ¹⁹⁵Sb, ¹⁹⁷Hg, ⁹⁷Ru, ¹⁰⁰Pd, ^{101m}Rh, and ²¹²Pb.

"Chemotherapeutic agent" as used herein includes, but is not limited to, methotrexate, daunomycin, mitomycin C, cisplatin, vincristine, epirubicin, fluorouracil, verapamil, cyclophosphamide, cytosine arabinoside, aminopterin, bleomycin, mitomycin C, democolcine, etoposide, mithramycin, chlorambucil, melphalan, daunorubicin, doxorubicin, tamosifen, paclitaxel, vincristin, vinblastine, camptothecin, actinomycin D, and cytarabine. Other examples are found in U.S. Patent Application Publication 2006/0121539 (Debinski et al.).

"Cytotoxic agent" or "toxic agent" as used herein includes, but is not limited to, maytansinoids and maytansinoid analogs, taxoids, CC-1065 and CC-1065 analogs, dolastatin and dolastatin analogs, ricin (or more particularly the ricin A chain), aclacinomycin, Diphtheria toxin, Monensin, Verrucarin A, Abrin, Tricothecenes, and Pseudomonas exotoxin A, taxol, cytochalasin B, gramicidin D, ethidium bromide, emetine, mitomycin, etoposide, tenoposide, anti-mitotic agents, such as the vinca alkaloids (e.g., vincristine and vinblastine), colchicin, anthracyclines, such as doxorubicin and daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof, antimetabolites (e.g., methotrexate, 6-mercaptopurine, 6-thioguanine, cytarabine, and 5-fluorouracil decarbazine), alkylating agents (e.g., mechlorethamine, thioepa chlorambucil, melphalan, carmustine (BSNU), lomustine (CCNU), cyclothosphamide, busulfan, dibromomannitol, streptozotocin, mitomycin C, and cis-dichlorodiamine platinum (II) (DDP)), and antibiotics, including, but not limited to, dactinomycin (formerly actinomycin), bleomycin, mithramycin, calicheamicin, and anthramycin (AMC)).

In some embodiments, cytotoxic agents include a Pseudomonas exotoxin or a Diphtheria toxin. See U.S. Patent No. 5,328,984 to Pastan et al. and U.S. Patent No. 6,296,843 to Debinski. Pseudomonas exotoxins can include, but are not limited to, Pseudomonas exotoxin A (PE). The Pseudomonas exotoxin can be modified such that it substantially lacks domain Ia, and in some embodiments Pseudomonas exotoxins include PE38QQR and PE4E. Diphtheria toxins can include DT390, a diphtheria toxin in which the native binding domain is eliminated. It will be appreciated that in various embodiments, the therapeutic agents can be attached to, e.g., the amino terminus or the carboxyl terminus.

"Detectable group" as used herein includes, but is not limited to, radiolabels (e.g., ³⁵S, ¹²⁵I, ³²P, ³H, ¹⁴C, ¹³¹I), enzyme labels (e.g., horseradish peroxidase, alkaline phosphatase), gold beads, chemiluminescence labels, ligands (e.g., biotin, digoxin) and/or fluorescence labels (e.g., rhodamine, phycoerythrin, fluorescein, fluorescent proteins), a fluorescent protein including, but not limited to, a green fluorescent protein or one of its many modified forms, a nucleic acid segment in accordance with known techniques, and energy absorbing and energy emitting agents.

The terms "treat," "treating" or "treatment" as used herein refers to any type of treatment that imparts a benefit to a patient afflicted with a disease, including improvement in the condition of the patient (*e.g*., in one or more symptoms), delay in the progression of the disease, etc.

The term "pharmaceutically acceptable" as used herein means that the compound or composition is suitable for administration to a subject to achieve the treatments described herein, without unduly deleterious side effects in light of the severity of the disease and necessity of the treatment.

"Concurrently administering" or "concurrently administer" as used herein means that the two or more compounds or compositions are administered closely enough in time to produce a combined effect (that is, concurrently may be simultaneously, or it may be two or more events occurring within a short time period before or after each other, e.g., sequentially). Simultaneous concurrent administration may be carried out by mixing the compounds prior to administration, or by administering the compounds at the same point in time but at different anatomic sites and/or by using different routes of administration.

The definitions and techniques described herein also apply to the IL13 proteins, toxin proteins, and other compounds and compositions mentioned hereinabove and hereinbelow.

### 2. Ephrin compounds.

Active compounds include ephrin molecules, e.g., ephrinA1, ephrinA2, ephrinA3, ephrinA4, ephrinA5, ephrinB1, ephrinB2, ephrinB3 proteins, peptides, variants, and fragments thereof. In particular embodiments, ephrin molecules are ephrinA1 proteins. In some embodiments, ephrin molecules are mammalian (e.g., human or mouse) ephrinA1 proteins. Further discussion of ephrin molecules, and their receptors, e.g., EphA2, is found in U.S. Patent Application Publication No. 2006/0121539 (Debinski et al.). In some embodiments, recombinant ephrinA1 includes a histidine tag for ease of purification.

EphrinA1 in dimeric form is known and may be from any (preferably mammalian) species, including, but not limited to, mouse, human, rat, dog, cat, monkey, etc. In some embodiments, the dimeric ephrinA1 includes the extracellular domain of ephrinA1 fused to the carboxy-terminal 6X histidine-tagged Fc region of human IgG via a polypeptide linker, as commercially available (e.g., Mouse Ephrin-A1/Fc Chimera, R&D Systems, Inc.).

In some embodiments of the invention, the ephrinA1 is provided in monomeric form. Active ephrinA1 in monomeric form is preferred over the currently-used covalently-linked dimeric form for its ease of use and design of ephrinA1-based therapies.

In one non-limiting embodiment of the invention, the amino acid/peptide sequence may be that of human ephrinA1 isoform a of SEQ ID: 2 (NCBI Accession No. AAH32698): or fragments or analogs thereof, encoded by the cDNA sequence of SEQ ID: 1 (NCBI Accession No. AAH32698). In non-limiting embodiments, a fragment of the peptide sequence starts at the aspartic acid residue at amino acid 19 (in bold). In further non-limiting embodiments, a fragment of the peptide sequence starts at the aspartic acid residue at amino acid 19, and ends at amino acid 182.

In another non-limiting embodiment of the invention, the amino acid/peptide sequence may be that of human ephrinA1 isoform a of **SEQ ID:** 4 (NCBI Accession No.NP_004419): or fragments or analogs thereof, encoded by the genomic DNA sequence of **SEQ ID:** 3 (NCBI Accession No. NM_004428). In non-limiting embodiments, a fragment of the peptide sequence starts at the aspartic acid residue at amino acid 19 (in bold). In further non-limiting embodiments, a fragment of the peptide sequence starts at the aspartic acid residue at amino acid 19, and ends at amino acid 182.

There is not 100% identity between the reported sequences of the human ephrinA1 isoform a genomic and cDNA clones. This results in a difference of 2 amino acid residues in the predicted protein sequences (underlined). The reported chimpanzee genomic sequence agrees with the human genomic sequence.

In another non-limiting embodiment of the invention, the amino acid/peptide sequence may be that of human ephrinA1 isoform b (NCBI Accession No. NP_872626) (**SEQ ID: 6**): or fragments or analogs thereof, encoded by **SEQ ID: 5** (NCBI Accession No. NM_182685). This isoform lacks a segment of 22 amino acids found in isoform a (residues 131-152). In non-limiting embodiments, a fragment of the peptide sequence starts at the aspartic acid residue at amino acid 19 (in bold). In further non-limiting embodiments, a fragment of the peptide sequence starts at the aspartic acid residue at amino acid 19, and ends at amino acid 160.

In a further non-limiting embodiment of the invention, the amino acid/peptide sequence may be that of murine ephrinA1 (NCBI Accession No. NP_034237): **(SEQ ID: 8)** or fragments or analogs thereof, encoded by the DNA sequence of **SEQ ID: 7** (NM_010107). In non-limiting embodiments, a fragment of the peptide sequence starts at the aspartic acid residue at amino acid 19 (in bold). In further non-limiting embodiments, a fragment of the peptide sequence starts with the aspartic acid at amino acid 19, and ends at amino acid 182.

Active compounds including small interfereing RNA (siRNA) are also contemplated herein (Landen Jr. et al. (2005) Cancer Res 65:6910-6918). Methods involving siRNA can be used to silence a targeted gene, e.g., an Eph receptor (such as EphA2).

### 3. Conjugates.

EphrinA1 induces EphA2 receptor internalization in addition to signaling through the receptor. This enables the utilization of ephrinA1 in the design of agents that require receptor-mediated internalization in order to be active, such as re-directed bacterial toxins (Debinski W. (2002) Molecular "Targeting of Brain Tumors with Cytotoxin," In: Chimeric Toxins (Lorberboum-Galski & Lazarovici, eds., Harwood Academic Publishers) pp. 222-246; Debinski (2002) Cancer Invest. 20:801-809). Accordingly, in some embodiments, ephrinA1 is provided in recombinant chimera constructs including cytotoxic proteins composed of ephrinA1 and modified proteinaceous bacterial toxins, which is a form of non-viral gene therapy. In this approach, it is expected that in one molecule of a drug candidate, a combined effect of receptor activation and a receptor-mediated drug delivery may work additively or even synergistically.

EphrinA1 in monomeric or covalently-linked dimeric form including, but not limited to, those described above, may be coupled to or conjugated to a therapeutic agent in accordance with any of a variety of techniques, such as those employed in the production of immunoconjugates. *See, e.g.,* U.S. Patent No. 6,949,245 to Sliwkowski.

In some embodiments, recombinant fusion chimera protein anti-cancer cytotoxins are composed of a carrier/ligand and an effector (catalyst). Carrier/ligands can be proteinaceous compounds, such as growth factors, cytokines, and monoclonal antibodies. Among effectors, bacterial toxins, such as Pseudomonas exotoxin A and Diphtheria toxin, or plant toxins, such as ricin may be utilized in some embodiments. The fusion protein is targeted only to cells expressing a target receptor/adaptor for a carrier/ligand. These targets internalize in response to carrier/ligand binding. Targets include, but are not limited to, protein receptors, antigens of various nature, adhesion molecules, gangliosides, etc. For example, EphA2 is over-expressed in a majority of patients with GBM and its ligand induces a receptor-mediated internalization once it binds the receptor. (Walker-Daniels et al. (2002) Mol Cancer Res 1:79-87). The latter may be used for, e.g., recombinant bacterial toxin-containing cytotoxins to exert anti-tumor action (Debinski W. (2002) Molecular "Targeting of Brain Tumors with Cytotoxin," In: Chimeric Toxins (Lorberboum-Galski & Lazarovici, eds., Harwood Academic Publishers) pp. 222-246; Debinski (2002) Cancer Invest. 20:801-809; Debinski (2002) Cancer Invest. 20:801-809).

Chemotherapeutic agents useful in the generation of such active compounds include those described above. Conjugates of ephrinA1 and one or more small molecule toxins, such as a calicheamicin, a maytansine (See U.S. Pat. No. 5,208,020), a trichothene, and CC 1065 are also contemplated herein. In some embodiments, conjugates of ephrinA1 to Pseudomonas exotoxins are used (U.S. Patent No. 5,328,984 to Pastan et al.).

In some embodiments of the invention, the ephrinA1 is conjugated to one or more maytansine molecules (e.g. about 1 to about 10 maytansine molecules per ephrinA1 molecule). Maytansine may, for example, be converted to May-SS-Me which may be reduced to May-SH3 and reacted with modified ephrinA1 (Chari et al. (1992) Cancer Research 52: 127-131) to generate an active compound.

Another conjugate of interest includes an ephrinA1 conjugated to one or more calicheamicin molecules. The calicheamicin family of antibiotics is capable of producing double-stranded DNA breaks at sub-picomolar concentrations. Structural analogues of calicheamicin that may be used include, but are not limited to, γ₁¹, α₂¹, α₃¹, N-acetyl-γ₁¹, PSAG and θ₁¹, (Hinman et al. (1993) Cancer Research 53:3336-3342; Lode et al. (1998) Cancer Research 58:2925-2928). See also U.S. Patent Nos. 5,714,586, 5,712,374, 5,264,586, and 5,773,001.

Enzymatically active toxins and fragments thereof which can be used are described above and include diphtheria A chain, nonbinding active fragments of diphtheria toxin, exotoxin A chain (from *Pseudomonas aeruginosa),* ricin A chain, abrin A chain (from *Corrybacterium typhimuriae),* modeccin A chain, alpha-sarcin, *Aleurites fordii* proteins, dianthin proteins, *Phytolaca americana* proteins (PAPI, PAPII, and PAP-S), *momordica charantia* inhibitor, curcin, crotin, sapaonaria officinalis inhibitor, gelonin, mitogellin, restrictocin, phenomycin, enomycin and the tricothecenes. See, for example, WO 93/21232 published Oct. 28, 1993.

The present invention further contemplates a conjugate formed between active compounds and an antibody or a compound with nucleolytic activity (e.g. a ribonuclease or a DNA endonuclease such as a deoxyribonuclease; DNase).

A variety of radioactive isotopes or radionuclides are available for the production of radioconjugated compounds as described above.

In some embodiments, conjugates of a monomeric ephrinA1 and therapeutic agents or detectable groups may be made using a variety of bi-functional protein coupling agents such as N-succinimidyl-3-(2-pyridyldithiol)propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis(p-azidobenzoyl)hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin conjugate can be prepared as described in Vitetta et al. (1987) Science 238:1098. Carbon-14-labeled 1-isothiocyanatobenzyl-3-methyldiethylene triaminepentaacetic acid (MX-DTPA) is an exemplary chelating agent for conjugation of radionucleotide to the ephrinA1 .See WO94/11026. The linker may be a "cleavable linker" facilitating release of the cytotoxic drug in the cell. For example, an acid-labile linker, peptidase-sensitive linker, dimethyl linker or disulfide-containing linker (Chari et al. (1992) Cancer Research 52:127-131) may be used.

Alternatively, a fusion protein including the ephrinA1 and therapeutic agent or detectable group may be made, e.g. by recombinant techniques or peptide synthesis.

In yet another embodiment, the ephrinA1 may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide).

Also contemplated herein are conjugates including ephrin mimetic peptides, as found in U.S. Patent Publication Nos. 2006/0177452 (Pasquale et al.) and 2004/0180823 (Pasquale et al.).

In some embodiments, the ephrinA1 includes amino acids 19-205 of the human ephrinA1, fused to a Pseudomonas exotoxin or Diptheria toxin. (U.S. Patent No. 5,328,984 to Pastan et al. and U.S. Patent No. 6,296,843 to Debinski). Pseudomonas exotoxins include, but are not limited to, Pseudomonas exotoxin A (PE). The Pseudomonas exotoxin can be modified such that it substantially lacks domain Ia, and Pseudomonas exotoxins may further include PE38QQR and PE4E. Diphtheria toxins include DT390, a diphtheria toxin in which the native binding domain is eliminated. It will be appreciated that the toxin can be connected to either of the amino terminus, e.g., DT390-ephrinA1 **(SEQ ID NO: 10),** or the carboxyl terminus, e.g., ephrinA1-PE38QQR **(SEQ ID NO: 12)** and ephrinA1-PE4E **(SEQ ID NO: 14).**

Another example of an embodiment of a structure of ephrinA1based cytotoxin is as follows: (I) ephrinA1 has an extension at its C-terminal end by a hinge region of human IgG only and a His-tag, and not the whole Fc-His as it is now used by commercial suppliers (the released ligand will be in a form of a homodimer, similarly to ephrinA-Fc-His), (II) nickel column-purified ephrinA1-hinge-His will be subjected to reducing conditions and freed sulfhydryl groups in the hinge region will be used for conjugation with maleimide-derivatized PE38QQR to form a covalent bond between the proteins, and (III) the ephrinA1-hinge-His-PE38QQR conjugate will be purified to isolate a monomer of ephrinA1-hinge-His-PE38QQR conjugate. In this conjugate, only one molecule of ephrinAl-hinge-His will be conjugated to one molecule of PE38QQR, since the singular reactive sites for conjugation will be in the hinge region of an engineered ephrinA1 and at the N-terminal end of PE38QQR. The size of this conjugate is ∼70 kDa. See also Debinski et al. (1992) Cancer Res 52:5379-5385. There are many alternative embodiments if further modification is desired. This includes the use of DT rather than PE toxin, adding hinge-CH2 to ephrinA1 (to increase the yield, if needed), generating monomers of ephrinA1 released from cells, and its derivatization with thiol-providing hetero-bifunctional cross-linkers as previously employed.

IL13-based conjugates are also contemplated herein. See, e.g., U.S. Patent Nos. 5,328,984 (Pastan et al.), 5,614,191 (Puri et al.), 5,919,456 (Puri et al.), 6,296,843 (Debinski), 6,428,788 (Debinski et al.), 6,518,061 (Puri et al.), 6,576,232 (Debinski et al.), 6,630,576 (Debinski), and 6,884,603 (Debinski et al.). The first generation of IL13-based cytotoxin, hIL13-PE38QQR, entered Phase III clinical trials in 2004 and is a Fast Track drug (Kunwar et al. (2003) Journal of Neurosurgery 98:697).

### 4. Pharmaceutical formulations.

The active compounds, conjugates, and/or compositions thereof described herein may be formulated for administration in a pharmaceutical carrier in accordance with known techniques. *See, e.g.,* Remington, The Science And Practice of Pharmacy (9th Ed. 1995). In the manufacture of a pharmaceutical formulation, the active compound (including the physiologically acceptable salts thereof) is typically admixed with, *inter alia*, an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.01 or 0.5% to 95% or 99% by weight of the active compound. One or more active compounds may be incorporated in the formulations, which may be prepared by any of the well known techniques of pharmacy comprising admixing the components, optionally including one or more accessory ingredients.

The formulations include those suitable for oral, rectal, topical, buccal (e.g., sub-lingual), vaginal, parenteral (e.g., subcutaneous, intramuscular, intradermal, or intravenous), topical (*i.e*., both skin and mucosal surfaces, including airway surfaces) and transdermal administration, although the most suitable route in any given case will depend on the nature and severity of the condition being treated and on the nature of the particular active compound which is being used.

Particular routes of parenteral administration include intrathecal injection, including directly into the tumor or a tumor resection cavity, and intraventricular injection into a ventricle of the brain.

Active compounds and compositions may be administered by intratumor injection (including tumors in any region such as tumors of the brain), or in the case of brain tumors injection into a ventricle of the brain.

Formulations suitable for parenteral administration comprise sterile aqueous and non-aqueous injection solutions of the active compound, which preparations are preferably isotonic with the blood of the intended recipient. These preparations may contain anti-oxidants, buffers, bacteriostats and solutes that render the formulation isotonic with the blood of the intended recipient. Aqueous and non-aqueous sterile suspensions may include suspending agents and thickening agents. The formulations may be presented in unit\dose or multi-dose containers, for example sealed ampoules and vials, and may be stored in a freeze-dried (lyophilized) condition requiring only the addition of the sterile liquid carrier, for example, saline or water-for-injection immediately prior to use. Extemporaneous injection solutions and suspensions may be prepared from sterile powders, granules and tablets of the kind previously described. For example, an injectable, stable, sterile composition may comprise an active compound or composition in a unit dosage form in a sealed container. The compound or composition is provided in the form of a lyophilizate that is capable of being reconstituted with a suitable pharmaceutically acceptable carrier to form a liquid composition suitable for injection thereof into a subject. The unit dosage form typically comprises from about 10 mg to about 10 grams of the compound or composition. When the compound or composition is substantially water-insoluble, a sufficient amount of emulsifying agent that is physiologically acceptable may be employed in sufficient quantity to emulsify the compound or composition in an aqueous carrier. One such useful emulsifying agent is phosphatidyl choline.

Further, described herein are liposomal formulations of the compounds disclosed herein and compositions thereof. The technology for forming liposomal suspensions is well known in the art. When the compound or composition thereof is an aqueous-soluble composition, using conventional liposome technology, the same may be incorporated into lipid vesicles. In such an instance, due to the water solubility of the compound or composition, the compound or composition will be substantially entrained within the hydrophilic center or core of the liposomes. The lipid layer employed may be of any conventional composition and may either contain cholesterol or may be cholesterol-free. When the compound or composition of interest is water-insoluble, again employing conventional liposome formation technology, the composition may be substantially entrained within the hydrophobic lipid bilayer that forms the structure of the liposome. In either instance, the liposomes that are produced may be reduced in size, as through the use of standard sonication and homogenization techniques.

Liposomal formulations containing the compounds disclosed herein or compositions thereof (e.g., ephrinA1 in monomeric form, or a conjugate thereof), may be lyophilized to produce a lyophilizate, which may be reconstituted with a pharmaceutically acceptable carrier, such as water, to regenerate a liposomal suspension. Examples of liposomal formulations that can be used include the neutral lipid 1,2-dioleoyl-*sn*-glycero-3-phosphatidylcholine (DPOC) (See, e.g., Landen Jr. et al. (2005) Cancer Res 65:6910-6918).

Other pharmaceutical compositions may be prepared from the water-insoluble compounds disclosed herein, or compositions thereof, such as aqueous base emulsions. In such an instance, the composition will contain a sufficient amount of pharmaceutically acceptable emulsifying agent to emulsify the desired amount of the compound or composition thereof. Particularly useful emulsifying agents include phosphatidyl cholines, and lecithin.

In addition to active compounds, the pharmaceutical compositions may contain other additives, such as pH-adjusting additives. In particular, useful pH-adjusting agents include acids, such as hydrochloric acid, bases or buffers, such as sodium lactate, sodium acetate, sodium phosphate, sodium citrate, sodium borate, or sodium gluconate. Further, the compositions may contain microbial preservatives. Useful microbial preservatives include methylparaben, propylparaben, and benzyl alcohol. The microbial preservative is typically employed when the formulation is placed in a vial designed for multidose use. Of course, as indicated, the pharmaceutical compositions of the present invention may be lyophilized using techniques well known in the art.

The therapeutically effective dosage of any one active agent, the use of which is in the scope of present invention, will vary somewhat from compound to compound, and patient to patient, and will depend upon factors such as the age and condition of the patient and the route of delivery. Such dosages can be determined in accordance with routine pharmacological procedures known to those skilled in the art.

As a general proposition, the initial pharmaceutically effective amount of the active compound or composition administered parenterally will be in the range of about 0.1 to 50 mg/kg of patient body weight per day, with the typical initial range of antibody used being 0.3 to 20 mg/kg/day, more preferably 0.3 to 15 mg/kg/day. The desired dosage can be delivered by a single bolus administration, by multiple bolus administrations, or by continuous infusion administration of active compound, depending on the pattern of pharmacokinetic decay that the practitioner wishes to achieve.

The active compound is suitably administered to the patient at one time or over a series of treatments. Depending on the type and severity of the disease, about 1 µg/kg to 15 mg/kg (e.g. 0.1-20 mg/kg) of active compound is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. The preferred dosage of the active compound will be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, e.g. about six doses of the anti-ErbB2 antibody). An initial higher loading dose, followed by one or more lower doses may be administered. An exemplary dosing regimen comprises administering an initial loading dose of about 4 mg/kg, followed by a weekly maintenance dose of about 2 mg/kg of the active compound. However, other dosage regimens may be useful. The progress of this therapy is easily monitored by conventional techniques and assays.

Subjects treated by the methods disclosed herein can also be administered one or more additional therapeutic agents. See U.S. Pat. No. 5,677,178. Chemotherapeutic agents may be administered by methods well known to the skilled practitioner, including systemically, direct injection into the cancer, or by localization at the site of the cancer by associating the desired chemotherapeutic agent with an appropriate slow release material or intra-arterial perfusing of the tumor. The preferred dose may be chosen by the practitioner based on the nature of the cancer to be treated, and other factors routinely considered in administering. See, e.g., US Patent No. 7,078,030.

Subjects may also be treated by radiation therapy, including, but not limited to, external beam radiotherapy, which may be at any suitable dose (*e.g.,* 20 to 70 Gy or more per tumor, typically delivered over a fractionated schedule).

Pharmaceutical compositions containing monomeric ephrinA1 in unlabeled form may be administered to subjects as blocking reagents, in like manner as described in Abrams et al., U.S. Patent No. RE38,008, in conjunction with the administration of monomeric ephrinA1 coupled to a therapeutic group

Monomeric ephrinA1 coupled to a diagnostic group may also be used *in vitro* as histological reagents on tissue samples, where binding of the ephrinA1 is indicative of cancer tissue in the tissue sample.

### 5. Combination therapies.

Growth factor or cytokine receptors on tumor cells may be attractive targets for tumor diagnosis, imaging, and therapy of gliomas provided that they offer widespread distribution, high levels of expression, and are specific for cancer cells (Debinski W. (2002) Molecular "Targeting of Brain Tumors with Cytotoxin," In: Chimeric Toxins (Lorberboum-Galski & Lazarovici, eds., Harwood Academic Publishers) pp. 222-246; Debinski (2002) Cancer Invest. 20:801-809; Debinski (2002) Cancer Invest. 20:801-809). We had previously discovered a unique molecular target that is richly over-expressed in malignant gliomas, and in HGGs in particular, that can be exploited uniquely for all three clinical purposes: diagnosis, imaging, and treatment. The target is a receptor (R) for the immune regulatory cytokine, interleukin 13 (IL13) (McKenzie et al. (1993) Proc. Natl. Acad. Sci. USA 90:3735-3739; Minty et al. (1993) Nature 362:248-251). The uniqueness of this cytokine is that its cancer-related receptor is present at limited amounts on several normal tissues. Furthermore, the binding to and physiological signaling by IL13 on normal cells and tissues is specifically achieved through a high-affinity, heterodimeric receptor complex, which it shares with IL4. Malignant glioma cells, on the other hand, express on their surfaces primarily a non-signaling monomeric form of the IL13R to which IL4 can neither bind nor transmit a signal.

This monomeric IL13 receptor is now a molecularly defined protein and termed IL13Rα2 (Mintz et al. (2002) Neoplasia 4:388-399). This restricted receptor is one of the first three factors ever documented to be expressed in a majority of patients with GBM and not in normal brain (Murphy et al. (1995) Gene 159:131-135; Rich et al. (1996) Gene 180:125-130). Numerous experiments further supported the notion that molecular targeting of IL13Rα2 is an attractive strategy for molecular detection and treatment of GBM. Targeted cytotoxic therapy, targeted gene therapy, targeted radiation therapy, and targeted chemotherapy all have the potential of being applied to patients with HGGs. Several therapeutic approaches are being developed with this target in mind, including vaccines (Okano et al. (2002) Clin Cancer Res 8:2851-2855; Mintz et al. (2002) Neuro-Oncology 4(4):334), targeted viruses (Zhou et al. (2002) Proc Natl Acad Sci 99:15124-15129), re-targeted cytotoxic T cells (Kahlon et al. (2004) Cancer Res 64:9160-9167) and new IL13-based cytotoxins (Li et al. (2002) Prot Engin 15:419-427; Mintz et al. (2003) J Neuro-Oncol 64:117-123). Thus, IL13Rα2 is a truly attractive molecular target.

The present invention is explained in greater detail in the following non-limiting Examples.

### EXAMPLES

**Examples 1-3.** We explored the role of ephrinA1 as a tumor-suppressing protein in GBM with respect to its impact on the level and activation status of EphA2, cell morphology and migration, and downstream signaling and anchorage-independent growth. We also investigated the form of ephrinA1 that fulfills these functions and are providing the first direct evidence that ephrinA1 exists in a soluble, monomeric form that is capable of eliciting a tumor-suppressing response in cancer cells that is not dependent on cell-cell contact. We provide evidence that the function of this monomeric form of ephrinA1 is not restricted to cancer cells, but extends to the physiological phenomena observed in the nervous system.

Western blotting and immunoprecipitation were performed as previously described (Wykosky et al. (2005) Mol. Cancer Res. 3:541-551). Conditioned media was collected and centrifuged for 5 min to remove non-soluble debris. Rabbit polyclonal anti-ephrinA1 (1:200) and anti-EphA2 (1:200) were purchased from Santa Cruz Biotechnology (Santa Cruz, CA). Mouse monoclonal EphA2 clone D7 (1:500), phosphotyrosine clone PY20 (1:1000) and β-action (1:50,000) antibodies were purchased from Sigma (St. Louis, MO). Rabbit polyclonal phospho-Erk (1:750) and total-Erk (1:1000) antibodies were purchased from Cell Signaling Technology, Inc. (Danvers, MA). Detection was performed using the ECL plus western Blotting Detection System (Amersham Biosciences, UK). Films were scanned at 600x dpi and images compiled using Jasc Paint Shop Pro v 6.0. Immunoprecipitation was performed as previously described (Wykosky et al. (2005) Mol. Cancer Res. 3:541-551), 500 µg of cell lysate was immunoprecipitated with 3 µg of monoclonal EphA2 (clone D7). Immunoprecipitates were collected and stored at -80 °C until separated by SDS-PAGE for western blotting.

Conditioned media was collected from sub-confluent monolayers of U-251 MG parental, vector-transfected, or *ephrinA1-* transfected cells. Media was centrifuged for 5 min at 1000 rpm to pellet any non-soluble debris, and supernatant was collected and used immediately or stored at -20 °C until use. Cells were treated with conditioned media, recombinant mouse ephrinA1/Fc chimera (R&D Systems) or mouse monoclonal IgG₁ isotype control (R&D Systems) for the indicated times. Cells were photographed by phase contrast microscopy and images were processed using Jasc Paint Shop Pro v. 6.01. Cell lysates were collected and used for western blotting and/or immunoprecipitation.

Immunofluorescence was performed as described previously (Wykosky et al. (2005) Mol. Cancer Res. 3:541-551). Primary antibodies EphA2 monoclonal (1:200) and ephrinA1 (1:200) were diluted in 1.5% NGS and incubated overnight at 4°C. Slides were washed twice in PBS and incubated with secondary antibody for 45 min at room temperature. Secondary antibodies included donkey anti-rabbit rhodamine (1:200) (Jackson ImmunoResearch Laboratories, Inc., West Grove, PA) or goat antimouse IgG Oregon Green (1:200) (Molecular Probes, Eugene, OR). Slides were counterstained with Hoescht No. 33258 Nuclear Counterstain (DAPI) (1:3000). Slides were mounted with Gel-Mount (Biomeda Corp., Foster City, CA).

For F-actin staining, cells were grown on sterile slides, treated with 1mg/mL ephrinA1-Fc or IgG-Fc isotype control for the desired time, then rinsed with PBS and fixed in 10% formalin for 5 min. Cells were permeabilized in PBS+0.1% Triton-X100 for 1 min, stained with AlexaFluor 488 phalloidin (1:200, Molecular Probes) and DAPI (1:3000) for 1 hr, rinsed in PBS, and mounted with Gel-Mount. All photomicrographs were taken with a 40x magnification oil immersion lens with a Zeiss Axiovision camera. Images were processed with Jasc Paint Shop Pro v6.01.

Statistical significance was determined by one-way analysis of variance (ANOVA). If p<0.05, Bonferroni's multiple comparison test was used to determine pair-wise significance in all cases with the exception of the neuronal growth cone analysis, in which Neuman-Keuls post-hoc testing was used. Error bars represent mean +/- S.E.M.

**Example 1: Transfection of U-251 MG GBM cells with ephrinA1.** GBM cell line U-251 MG was obtained from the American Type Culture Collection (Manassas, VA). U-251 MG cells were grown in DMEM+glutamine with 10% FBS and 0.1 mmol/L non-essential amino acids. DNA (5µg; *ephrinA1*pcDNA*, His-ephrinA1* pcDNA or empty vector control) was transfected into cells growing in Opti-MEM (Invitrogen, Carlsbad, CA) using LipofectAMINE 2000 (Invitrogen). After 24 hrs, Opti-MEM was replaced with growth medium containing 20% fetal bovine serum. 24 hr later, the cells were split into 100 mm² Petri dishes and geneticin (800 µg/mL) was added to select clones. Individual clones were isolated and maintained in appropriate growth medium containing 200 µg/mL geneticin.

To examine what form of ephrinA1 fulfills a tumor suppressor function, we transfected U-251 MG GBM cells with *ephrinA1* and observed diminished EphA2 levels when the cells were confluent; this supported the membrane-anchored presence of ephrinA1 and cell-to-cell interaction responsible for EphA2 activation and ensuing degradation. Unexpectedly, when cells were not confluent, EphA2 became lower, too, suggesting a release of a monomeric full-length ephrinA1 corresponding to the gene used for transfection.

Next, we detected a monomer of ephrinA1 in the media of the U-251 MG-ephrinA1(+) cells, but not in the media of control cells. We also found that the media of the U-251 MG-ephrinA1(+) cells contained the EphA2-decreasing activity. In addition, the media caused EphA2 phosphorylation in a dose-dependent manner confirming specific activation of the EphA2 receptor. Moreover, a previously observed ability of recombinant ephrinA1-Fc to induce profound morphological changes in GBM cells was detected in the media of U-251 MG-ephrinA1(+) cells as well.

**A. EphrinA1-producing GBM cells and breast cancer cells exhibit down-regulated EphA2 independent of cell-cell contact.** To investigate the functional interplay between EphA2 and ephrinA1, U-251 MG GBM cells, which naturally express high levels of EphA2 and very low levels of ephrinA1, were stably transfected with full-length human *ephrinA1* **(SEQ ID: 1).** Total RNA was isolated from human umbilical vein endothelial cells and reverse transcription was used to generate corresponding cDNA. The DNA sequence corresponding to the full-length human *ephrinA1* gene was amplified by PCR using oligonucleotide primers specific for the sequence of human *ephrinA1.* The *ephrinA1* gene product was then cloned into a commercially available plasmid mammalian expression vector, pcDNA 3.1(+) (Invitrogen), at the multi-cloning site of this vector.

Western blotting revealed that three clonally selected lines (#4, #7, and #12) over-expressed ephrinA1, which migrated as an immunoreactive band of ∼25 kDa **(****Figure 1A****).** Interestingly, we observed a dramatic decrease in EphA2 protein in those cells harboring the *ephrinA1* transgene, in comparison with high levels of EphA2 in parental and mock-transfected cells **(****Figure 1A****).** Immunofluorescence revealed the same differential expression of ephrinA1 and EphA2 (data not shown). Notably, ephrinA1-specific staining in transfected cells was more diffusely cytoplasmic and perinuclear and thus different from the staining pattern seen for EphA2, suggesting that ephrinA1in these cells is not a membrane-localized protein. Hence, GBM cells that normally express high levels of EphA2 and low ephrinA1 can be stably transfected to over-express ephrinA1 that does not localize to cell membranes, and this coincides with a marked decrease in plasma membrane-bound EphA2.

EphA2 undergoes ligand-mediated receptor internalization and degradation following activation by recombinant, homodimeric ephrinA1-Fc (Walker-Daniels et al. (2002) Mol. Cancer Res. 1:79-87). We investigated if the down-regulation of EphA2 in U-251 [*ephrinA1*](+) cells is dependent on the extent of cell-cell contact, and potentially due to a membrane-bound form of ephrinA1. Thus, *ephrinA1*(+) and mock-transfected U-251 MG cells were plated at low density or grown to confluency **(****Figure 1B****).** Also examined were SK-BR-3 cells, a breast carcinoma cell line shown previously to express high endogenous ephrinA1 and low EphA2 in cell lysates (Macrae et al. (2005) Cancer Cell 8:111-118). Breast cancer cell line SK-BR-3 weas obtained from the American Type Culture Collection (Manassas, VA). SK-BR-3 cells were grown in RPMI with 10% FBS.

The down-regulation of EphA2 in U-251 [*ephrinA*1](+) cells persisted despite the lack of extensive cell-cell contact **(****Figure 1C****).** Furthermore, SK-BR-3 cells displayed undetectable levels of EphA2 at both low and high density. These findings support the notion that both ectopic and endogenous ephrinA1 is capable of down-regulating EphA2 in a manner that is not dependent on cell-cell contact.

**B. EphrinA1 is a soluble, monomeric protein in the media of cancer cells.** To initially investigate the possible existence of soluble **ephrinA1**, we tested media from U-251 [*ephrinA1*](+) and SK-BR-3 cells. Western blot analysis for ephrinA1 under reducing conditions revealed abundant expression of the protein in the media of *ephrinA1*-transfected and SK-BR-3 cells, but not parental or mock-transfected cells **(****Figure 2A****).** To characterize the form of ephrinA1 in the media of transfected cells, we performed western blotting under non-reducing conditions, in which we detected a single, 25-kDa immunoreactive band corresponding to monomeric ephrinA1 in the media of the two studied U-251 [*ephrinA1*](+) clones: #4 and #7 **(****Figure 2B****).** There were no ephrinA1-immunoreactive proteins detected at the expected size of an ephrinA1 homodimer (50-60 kDa), suggesting that ephrinA1 is present as a monomer in the media of these cells. The same results were obtained with SK-BR-3 media under non-reducing conditions, suggesting that ephrinA1 is also a monomeric protein in cells that naturally produce the ligand (data not shown).

**C. Monomeric, soluble ephrinA1 alters GBM cell morphology and down-regulates the EphA2 oncoprotein.** Activation of EphA2 with recombinant homodimeric **ephrinA1-Fc** induces rapid, profound changes in the actin cytoskeleton in addition to causing receptor internalization and degradation (Miao et al. (2000) Nat. Cell Biol. 2:62-69). To explore the functional activity of soluble monomeric ephrinA1, parental U-251 MG cells were treated with media from self, mock-transfected, or U-251 [*ephrinA1*](+) cells. Cells treated with conditioned media from control cells retained the shape typical of U-251 cells with distinct, elongated processes **(****Figure 3A****).** However, cells treated with conditioned media from three different ephrinA1(+) clones ("ephrinAl-conditioned media") rapidly became rounded, retracting most or all processes within 30 min **(****Figure 3A****).** This phenomenon was dose-dependent, since its extent corresponded to the amount of conditioned media applied **(****Figure 3B****).** We observed similar U-251 MG cell rounding in response to treatment with recombinant dimeric ephrinA1-Fc **(****Figure 3C****).**

To validate the existence of functional, soluble ephrinA1 in the media of transfected cells, we investigated the effect of ephrinA1-conditioned media on the expression of EphA2. Treatment of parental U-251 MG cells not harboring an *ephrinA1* transgene with ephrinA1-conditioned media caused significant down-regulation of the receptor **(****Figure 4A****).** The magnitude of EphA2 down-regulation in response to treatment with ephrinA1-conditioned media compared to treatment with ephrinA1-Fc was similar, although EphA2 expression began to decline after 60 min in response to ephrinA1-conditioned media and after 30 min in response to ephrinA1-Fc **(****Figure 4B****).** In both cases, EphA2 remained suppressed for at least 24 hr in the presence of the ligand. Cells treated with parental or mock-conditioned media over time exhibited no change in the level of EphA2 whatsoever (data not shown). These results indicate that a soluble form of ephrinA1 that is present in the conditioned media of U-251 [*ephrinA1*](+) cells has the ability to .function in a paracrine fashion on the EphA2 receptor.

**D. Soluble, monomeric ephrinA1 negatively regulates the Ras-MAPK pathway and suppresses anchorage-independent growth.** Activation of EphA2 by ephrinA1 results in a suppression of signaling through the oncogenic Ras-MAPK pathway in breast and prostate cancer cells as well as in keratinocytes isolated from mouse skin (Miao et al. (2001) Nat. Cell Biol. 3:527-530; Guo et al. (2006) Cancer Res. 66:7050-7058). We investigated the effect of both ephrinA1-Fc- and ephrinA1-conditioned media on Ras-MAPK signaling downstream of EphA2 in GBM. At 10 min following treatment of U-251 MG cells with ephrinA1-conditioned media, we detected a sharp decrease in p-ERK expression **(****Figure 5A****).** p-ERK remained suppressed up to 24 hr, at which point it had returned to baseline levels. Treatment with ephrinA1-Fc resulted in similar suppression of p-ERK, but was observed later, at about 1 hr following stimulation, and persisted through 24 hr **(****Figure 5B****).** These observations suggest a prominent inhibition of the Ras-MAPK pathway by monomeric ephrinA1 through EphA2.

In another attempt to document a tumor-suppressing role for soluble, monomeric ephrinA1, U-251 MG cells expressing the ligand were assessed for its effect on anchorage-independent growth. U-251 [*ephrinA1]*(+) cells (#4 and #12) displayed a dramatic impairment in the ability to form colonies in soft agar when compared to parental and mock-transfected cells (p<0.001) **(****Figure 5C****).** Notably, the defect in anchorage-independent growth was similar for ephrinA1-producing cells and controls treated with dimeric ephrinA1-Fc.

**Example 2: Transfection of U-251 MG GBM cells with His-ephrinA1.** In another direct experiment to investigate the function of monomeric ephrinA1, we transfected U-251 MG cells with N-terminal 6-histidine-tagged ephrinA1 (His-ephrinAl). U-251 MG cells transfected with *His-ephrinA1* were grown in 150 cm² tissue culture flasks until sub-confluent. Normal culture medium was replaced with low-serum medium containing 1% FBS for 24 hours. Conditioned media was collected and subject to buffer exchange by dialysis with binding buffer (20mM sodium phosphate, 0.5M sodium chloride, 20mM imidazole, pH 7.4). Dialyzed conditioned media was filtered and His-ephrinA1 purified by HisTrapHP Ni Sepharose High Performance via FPLC (Amersham, Piscataway, NJ). Purified protein was eluted from column with binding buffer containing 500mM imidazole. Protein was concentrated and buffer was exchanged to PBS with Microcon centrifugal filter devices (Millipore, Bedford, MA).

His-ephrinA1 was present as a monomer in the media of these cells and not in parental or mock-transfected cells, as seen by ephrinA1 western blotting under non-reducing conditions with two clonally selected lines **(****Figure 6E****).** The monomeric protein was isolated from the media via Ni²⁺ affinity chromatography, and was immunoreactive for ephrinA1 at the expected size of 25 kDa **(****Figure 6E****).** This purified protein was then used to treat U-251 MG cells, which responded to the monomeric ligand by changing cell morphology in a manner identical to ephrinA1-conditioned media and ephrinA1-Fc monomer or dimer **(****Figure 6F****).**

Together, these results reveal that covalent dimerization and/or artificial clustering of ephrinA1 is not required for EphA2 activation or for the downstream phenotypic effects of cell rounding and migration and support the notion that soluble monomeric ephrinA1 released into the extracellular environment is a functional protein with tumor-suppressing effects on cancer cells via the EphA2 receptor.

**Example 3: Reduction of a Homodimer of Recombinant ephrinA1-Fc.** In another direct experiment, we reduced a homodimer of recombinant ephrinA1-Fc (R&D Systems) and blocked the reactive sulfhydryl groups. 10mM DTT (Acros Organics, Morris Plains, NJ) was added to recombinant mouse ephrinA1-Fc or IgG₁ isotype control diluted in PBS for 15 min at 37 °C. Iodoacetamide (IA) (Sigma) was added at a final concentration of 20mM and incubated at room temperature for 20 min. 10mM DTT was added again and incubated for 15 min at 37 °C to scavenge any unused IA. Reduced products were used to treat cells, were subject to purification using Spin-OUT 12,000 Micro columns (Chemicon, Temecula, CA), or were visualized by separation with SDS-PAGE followed by staining with Coomassie blue.

Our finding that soluble ephrinA1 in conditioned media exists as a functional, monomeric protein led us to investigate the possibility that recombinant ephrinA1-Fc (R&D Systems) may be functional in a monomeric form as well. Thus, the disulfide bonds in ephrinA1-Fc were reduced, and free thiol groups were covalently modified by iodoacetamide (IA) (Debinski et al. (1992) J. Clin. Invest 90:405-411). SDS-PAGE analysis of reduced and non-reduced proteins verified the presence of monomeric or dimeric ephrinA1-Fc, respectively **(****Figure 6A****).**

Strikingly, both dimeric and monomeric ephrinA1-Fc were capable of activating the receptor, as revealed by the presence of the immunoreactive band corresponding to phosphorylated EphA2 **(****Figure 6B****).** The duration of EphA2 phosphorylation in response to treatment with the ephrinA1-Fc monomer was even longer than with the dimer. Phosphorylation of EphA2 was specifically mediated by ephrinA1-Fc and not affected by treatment with reducing agent and **IA**, or reduced and non-reduced IgG. Furthermore, treatment of U-251 MG cells with both dimeric and monomeric ephrinA1-Fc, and not IgG or PBS, resulted in significant cell rounding and loss of polarity **(****Figure 6C****).**

**A. Anchorage-independent growth assay.** 2 x 10³ cells were plated in 6-well dishes in growth medium plus 0.35% Agar (Difco), on a base layer of growth medium plus 0.5% Agar. Cells were supplemented with 1.0 µg/mL ephrinA1-Fc. ephrinA1-Fc was replenished and cells supplemented with fresh media 3 days after plating, and colonies were counted and photographed at low power after 14 days. Clusters of colonies greater than ∼50 cells were counted in ten random fields at low power; each experimental condition or cell line was assayed in triplicate.

**B. Migration assay.** Further supporting the function of a monomer of ephrinA1, both monomeric and dimeric ephrinA1-Fc were very effective in inhibiting the migration of U-251 MG in a wound closure assay (p<0.001 *vs.* IgG-treated control) **(****Figure 6D****).** U-251 MG cells were seeded onto 6-well dishes and allowed to grow until ∼95% confluent. Wounds were made with a sterile 200 µL tip, cells were washed with PBS, and growth media containing monomeric or dimeric ephrinA1-Fc (1 µg/mL) was added. Phase contrast-microscopy pictures were taken of the same field at 0 hr and 24 hr following addition of treatment. ImagePro Plus software was used to analyze images. Distance of the wound in µM was measured in three places for each wound at each time point, and the percent wound closure over 24 hr was calculated for graphical representation. Thus, a monomer of ephrinA1-Fc has a similar potency to the homodimer in activating EphA2 in GBM cells.

**C. Axonal Growth Cone Collapse.** To investigate the possibility that a monomer of ephrinA1 is functional in mediating normal physiological processes, we compared the role of monomeric and dimeric ephrinA1-Fc in axonal growth cone collapse. Primary rat cortical neurons were treated with monomeric or dimeric ephrinA1-Fc and fluorescently stained for F-actin to visualize growth cone structures.

Primary neuronal cultures were prepared as previously described (Turner et al. (2002) Exp. Neurol. 178:21-32). Briefly, the cortical lobes of embryonic day 18 Sprague-Dawley rat embryos were dissected and cells plated at low density on coverslips coated with 20 µg/mL poly-D-lysine (Sigma) and grown for 24 hours. EphrinA1-Fc (1 and 5 µg/mL), PBS (equal volume to ephrinA1-Fc), or IgG₁ isotype control (5 µg/mL) was added and cells incubated for 1 hour at 37 °C. Cells were fixed, permeabilized, and stained with rhodamine-phalloidin (Invitrogen) for 20 min at room temperature, washed, and coverslips were mounted onto glass slides using Vectashield Hard Set mounting media containing DAPI (Vector Labs, Burlingame, CA). Images were acquired using an Olympus IX70 Inverted System Microscope (Olympus, Melville, NY) and Hamamatsu digital camera (Hamamatsu City, Japan) with IPLab 3.6.4 software (Scanalytics, Inc, Fairfax VA). Image-Pro Plus v.5.1 software was used for image analysis. Collapsed growth cones (CGC) were categorized as having a shrunken lamellipodia with no filopodia, and counts were normalized to the number of DAPI-stained nuclei in the same field by expressing data as a ratio of the number of collapsed growth cones/number of nuclei (CGC/DAPI). Mean CGC/DAPI ratio was determined for each treatment group.

Treatment with ephrinA1-Fc in both a monomeric and dimeric form for 1 hr resulted in a significant increase in collapsed axonal growth cones (p<0.001 versus vehicle-treated control) **(****Figure 7****).** We did not observe significantly more growth cone collapse in response to treatment with 5 µg/mL of ligand, suggesting that a concentration of 1 µg/mL of ephrinA1-Fc represents saturating conditions. In sharp contrast to the distinct, collapsed growth cones of neurons treated with monomeric or dimeric ephrinA1-Fc, non-treated, vehicle-treated, or those neurons treated with IgG displayed well-developed, expanded growth cone structures and few, if any, collapsed growth cones (data not shown).

**Example 4: Creation of ephrinA1-cytotoxin constructs.** Being that EphA2 is an internalized receptor that is over-expressed in GBM cells, we have produced a novel cytotoxin composed of ephrinA1 a ligand for EphA2, and a genetically modified bacterial toxin, *Pseudomonas* exotoxin A (PE38QQR) **(SEQ ID: 12).**

EphrinA1-PE38QQR exhibited extremely potent and dose-dependent killing of GBM cells expressing EphA2, with an average IC₅₀ of ∼ 10⁻¹¹ M using a cell viability assay. The cytotoxic effect of ephrinA1-PE38QQR was specific, since it was completely neutralized by an excess of ephrinA1 ligand. Cells that do not over-express EphA2, including normal human endothelial cells and GBM cells with down-regulated EphA2, were not responsive to the cytotoxin. Notably, ephrinA1-PE38QQR was also effective against breast and prostate cancer cells that over-express the EphA2 receptor.

To further validate the potential usefulness of EphA2-targeted therapies such as ephrinA1-PE38QQR, we explored the presence and localization of EphA2 in GBM. We employed tissue micro-arrays containing various grades of astrocytomas and normal brain and performed immunohistochemistry (IH). We detected that the level of EphA2 expression is significantly elevated in GBM versus lower-grade astrocytomas and normal brain (p=0.001 GBM vs. normal brain, p=0.01 GBM *vs.* grade II or grade III). EphA2 is localized specifically on the surface of GBM cells, both in established cell lines and *in situ,* as revealed by flow cytometry, confocal microscopy, and IH. Importantly, EphA2 is absent in normal brain.

We cloned the cDNA encoding for a DT toxin, DT390, connected to ephrinA1 **(SEQ ID: 9).** The protein DT390-ephrinA1 **(SEQ ID: 10)** begins with the DT sequence at the N-terminal end of the fusion protein. The N-terminal end of ephrinA1 is linked to the toxin, and the C-terminal end of the construct is a free C-terminal end of ephrinA1 We have also cloned ephrinA1-PE4E **(SEQ ID: 13; SEQ ID: 14).**

We have expressed all three cytotoxin constructs in *E. coli* and obtained the best expression levels thus far for ephrinA1 and DT390-ephrinA1 **(****Figures 8A-8B****).** Therefore, we grew a larger scale culture of bacteria transformed with the DT390-ephrinA1 and localized the expressed protein to the inclusion bodies. We then isolated the inclusion bodies, denatured and renatured the protein, and purified using FPLC. We have tested the partially purified fractions of DT390-ephrinA1 in a cell proliferation assay using the U-251 MG GBM cells and were surprised to see that at 15 nM concentration, the cytotoxin started to kill cancer cells. In order to document the specificity of the killing, we pre-treated cells with commercially available ephrinA1-IgG recombinant protein and we observed that this protected cancer cells against the cytotoxic action of DT390-ephrinA1 **(****Figure 8A****).**

It is noteworthy that in the ephrinA1-IgG, ephrinA1 is placed at the N-terminal end of the protein. We had not expected DT390-ephrinA1 to exert any of the cytotoxic activity based on previous information that ephrinA1 monomer is potentially a much weaker ligand than its dimer, and that the EphA2 dimerization is needed for receptor internalization. Also, it is likely that either the N- or C-terminal end of ephrinA1 can be linked to other recombinant proteins, the property that we found attributable to IL13 as a receptor ligand as well.

**Example 5: Transient silencing of EphA2 and/or ephrinA1 with siRNA.** A new technology has been developed in which mammalian genes can be knocked down by transient transfection with silencing RNA duplexes (siRNA). We have started to employ this methodology while successfully silencing an AP-1 transcription factor, Fra-1; we discovered this factor to be highly up-regulated in GBM (Debinski et al. (2001) Mol Med 7:598-608).

We designed the siRNA sequence to target human EphA2:
sense 5' **UGAAUGACAUGCCGAUCUA 3' (SEQ ID: 15)** and
anti-sense 5' **UAGAUCGGCAUGUCAUUCA 3' (SEQ ID: 16).**
This sequence was BLAST-searched against expressed sequence tag libraries to ensure the specificity of the siRNA molecule. Desalted and deprotected synthetic oligonucleotides are made by Dharmacon (LaFayette, CO). The annealing of oligonucletides is performed in 100 mM potassium actetate, 30 mM HEPES-KOH, pH 7.4, 2 mM magnesium acetate for 1 min. at 90 °C, followed by 1 hr at 37 °C. Control duplex oligonucleotides correspond to the inverse sequence or an siRNA scramble duplex. siRNAs are transfected using the OligofectAMINE reagent (Invitrogen Life technologies). Transient transfections are carried out on 40-50% confluent cells plated one day before the experiment with 10% FBS without antibiotics using the siRNA *ephA2* duplex and corresponding controls, in at least duplicates. For one well of a 24-well plate, 0.84 µg siRNA duplex is used. The effect of silencing is assayed 2, 3, 5 and 7 days after transfection. It has been observed that the silencing using siRNAs lasts between 6 and 10 generation times. The transfection efficiency will be evaluated by western blotting of EphA2 and general cell morphology, and compared to stably transfected and control cell lines.

We have performed pilot experiments in order to verify the silencing of EphA2 in U-251 MG GBM cells and found at least 50% decrease in immunoreactive EphA2 when standardized versus actin control **(****Figure 9****).** In addition, using these siRNA resulted in a similar degree of EphA2 decrease in cells, producing a prominent anti-tumor effect *in vivo,* and thus represents a molecular candidate drug in tumors over-expressing EphA2, such as ovarian carcinoma and pancreatic adenocarcinoma (Landen et al. (2005) Cancer Res 65:6910-6818; Duxbury et al. (2004) Oncogene 23:1448-1456).

**Example 6: Evaluation of Combination Therapies for Patients with GBM.** A single molecular target may not be sufficient for creating a therapy that will be effective against all patients with GBM. Therefore, we investigated the expression of EphA2 versus IL-13Rα2, a receptor for IL-13 that our laboratory had previously identified and against which we have created potent recombinant cytotoxins that are in phase I through III clinical trials. Immunohistochemistry revealed that nearly 100% of GBM specimens tested expressed at least one of these molecular targets.

**Example 7: Molecular targeting of EphA2 and IL13R to xenografts.** Three types of human xenografts of non-established GBM cells were obtained that can be implanted into immunocompromised animals. These three types of human GBM explants were characterized for EGFR expression: Mayo-GBM-5 does not demonstrate EGFR amplification, Mayo-GBM-6 has the EGFRvIII mutant amplified, and Mayo-GBM-12 has the wild type EGFR amplified (Pandita et al. (2004) Genes Chromosomes Cancer 39:29-36). These explants produce intracranial tumors of infiltrative character, a trait not seen with the majority of established human GBM cell lines (Pandita et al. (2004) Genes Chromosomes Cancer 39:29-36). We have treated GBM-Mayo-6 and Mayo-GBM-5 cells with an IL13-basedcytotoxin, IL13.E13K-PE38QQR, and found that they are highly responsive to the cytotoxin. In fact, they appear to be more susceptible to the cytotoxin than the U-251 MG cells considered to be high-responders to the IL13 cytotoxins (e.g., Debinski et al. (1999) Clin Cancer Res 5:985-990). The IC₅₀ in Mayo-GBM6 and MayoGBM5 cells is 0.3 ng/mL and 0.2 ng/mL, respectively, while in U-251 MG cells it was 0.8 to 1.5 ng/mL in two independent assays. Thus, these explants are several times more responsive towards the cytotoxin than the established GBM cells, which is indicative of a high level of expression of IL13Rα2. Interestingly, these cells over-express EphA2 and are suitable for *in vivo* targeted anti-EphA2 therapies.

This is the first attempt to create a cytotoxic therapy based on any of the ephrin ligands of either class (A or B). EphrinA1-PE38QQR **(SEQ ID: 12)** is potent and specific, and forms the basis for the further clinical development of ephrinA1-based cytotoxins that can also be used in combination with IL13Rα2-directed therapies to improve the outcome of patients with GBM and other EphA2-expressing tumors.

The foregoing is illustrative of the present invention, and is not to be construed as limiting thereof. The invention is defined by the following claims, with equivalents of the claims to be included therein.

## Claims

1. EphrinA1 in monomeric form for use in the treatment of a cancer expressing the oncogenic receptor EphA2.

2. EphrinA1 in monomeric form for use in the treatment of cancer expressing the oncogenic receptor EphA2 according to claim 1, wherein said EphrinA1 is coupled to a detectable group or a therapeutic agent.

3. EphrinA1 in monomeric form for use in the treatment of cancer expressing the oncogenic receptor EphA2 according to claim 2, wherein said therapeutic agent is a radionuclide, chemotherapeutic agent or cytotoxic agent.

4. EphrinA1 in monomeric form for use in the treatment of cancer expressing the oncogenic receptor EphA2 according to claim 2, wherein said therapeutic agent comprises a Pseudomonas exotoxin.

5. EphrinA1 in monomeric form for use in the treatment of cancer expressing the oncogenic receptor EphA2 according to claim 2, wherein said detectable group comprises an enzyme label, gold bead, chemiiuminescent label, ligand or fluorescent label.

6. EphrinA1 in monomeric form for use in the treatment of cancer expressing the oncogenic receptor EphA2 according to claim 1 or 2, wherein said cancer is selected from the group consisting of breast cancer bladder cancer, pancreatic cancer, and glioma.

7. EphrinA1 in monomeric form for use in the treatment of cancer expressing the oncogenic receptor EphA2 according to claim 1, wherein the EphrinA1 in monomeric form further comprises IL-13 or a mutant thereof,

## Patentansprüche

1. EphrinA1 in monomerer Form zur Verwendung in der Behandlung eines den onkogenen Rezeptor EphA2 exprimierenden Krebses.

2. EphrinA1 in monomerer Form zur Verwendung in der Behandlung von den onkogenen Rezeptor EphA2 exprimierendem Krebs nach Anspruch 1, wobei das EphrinA1 an eine nachweisbare Gruppe oder ein therapeutisches Mittel gekoppelt ist.

3. EphrinA1 in monomerer Form zur Verwendung in der Behandlung von den onkogenen Rezeptor EphA2 exprimierendem Krebs nach Anspruch 2, wobei das therapeutische Mittel ein Radionuklid, ein chemotherapeutisches Mittel oder ein zytotoxisches Mittel ist.

4. EphrinA1 in monomerer Form zur Verwendung in der Behandlung von den onkogenen Rezeptor EphA2 exprimierendem Krebs nach Anspruch 2, wobei das therapeutische Mittel ein Pseudomonas-Exotoxin umfasst.

5. EphrinA1 in monomerer Form zur Verwendung in der Behandlung von den onkogenen Rezeptor EphA2 exprimierendem Krebs nach Anspruch 2, wobei die nachweisbare Gruppe eine Enzymmarkierung, ein Goldkügelchen, eine chemilumineszente Markierung, einen Liganden oder eine fluoreszierende Markierung umfasst.

6. EphrinA1 in monomerer Form zur Verwendung in der Behandlung von den onkogenen Rezeptor EphA2 exprimierendem Krebs nach Anspruch 1 oder 2, wobei der Krebs aus der Gruppe, bestehend aus Brustkrebs, Blasenkrebs, Bauchspeicheldrüsenkrebs und Gliom, ausgewählt ist.

7. EphrinA1 in monomerer Form zur Verwendung in der Behandlung von den onkogenen Rezeptor EphA2 exprimierendem Krebs nach Anspruch 1, wobei das EphrinA1 in monomerer Form weiterhin IL-13 oder einen Mutanten davon umfasst.

## Revendications

1. Ephrine-A1 sous forme de monomère à utiliser dans le traitement d'un cancer exprimant le récepteur oncogénique EphA2.

2. Ephrine-A1 sous forme de monomère à utiliser dans le traitement d'un cancer exprimant le récepteur oncogénique EphA2 selon la revendication 1, où ladite Ephrine-A1 est couplée à un groupe détectable ou à un agent thérapeutique.

3. Ephrine-A1 sous forme de monomère à utiliser dans le traitement d'un cancer exprimant le récepteur oncogénique EphA2 selon la revendication 2, où ledit agent thérapeutique est un radionuclide, un agent chimiothérapeutique ou un agent cytotoxique.

4. Ephrine-A1 sous forme de monomère à utiliser dans le traitement d'un cancer exprimant le récepteur oncogénique EphA2 selon la revendication 2, où ledit agent thérapeutique comprend une exotoxine de *Pseudomonas.*

5. Ephrine-A1 sous forme de monomère à utiliser dans le traitement d'un cancer exprimant le récepteur oncogénique EphA2 selon la revendication 2, où ledit groupe détectable comprend un marqueur enzymatique, une bille d'or, un marqueur chimiluminescent, un ligand ou un marqueur fluorescent.

6. Ephrine-A1 sous forme de monomère à utiliser dans le traitement d'un cancer exprimant le récepteur oncogénique EphA2 selon la revendication 1 ou 2, où ledit cancer est sélectionné parmi le groupe consistant en cancer du sein, cancer de la vessie, cancer du pancréas et gliome.

7. Ephrine-A1 sous forme de monomère à utiliser dans le traitement d'un cancer exprimant le récepteur oncogénique EphA2 selon la revendication 1, où l'Ephrine-A1 sous forme de monomère comprend en outre une IL-13 ou un mutant de celle-ci.
